# EUROPÄISCHE PATENTANMELDUNG

(11) **EP 4 431 612 A1**
(43) Veröffentlichungstag der Anmeldung: **18.09.2024**
(21) Anmeldenummer: 23162704.3
(22) Anmeldetag: 17.03.2023
(51) Int. Cl.: C12P 17/12, C12P 15/00, C07C 231/02, C07C 233/08, C07D 221/02

(54) **VERFAHREN ZUR HERSTELLUNG VON CORYTUBERIN UND SALUTARIDIN**

(71) Anmelder: Universität Graz, 8010 Graz (AT)
(72) Erfinder: Kroutil, Wolfgang, 8010 Graz (AT); Pletz, Jakob, 8010 Graz (AT); Cigan, Emmanuel, 8010 Graz (AT)
(74) Vertreter: Ellmeyer, Wolfgang

(57) **Zusammenfassung**

Die Erfindung betrifft ein Verfahren zur Herstellung von Corytuberin und Salutaridin über Reticulin als Zwischenprodukt, wobei das Verfahren die folgenden Reaktionsschritte umfasst:
a) Amidierung von 2-(4-Hydroxy-3-methoxyphenyl)-N-methylethanamin (12) mit 3-Hydroxy-4-methoxyphenylessigsäure zum N-[2-(4-Hydroxy-3-methoxyphenyl)ethyl-N-methyl-3-hydroxy-4-methoxyphenylessigsäureamid (13): 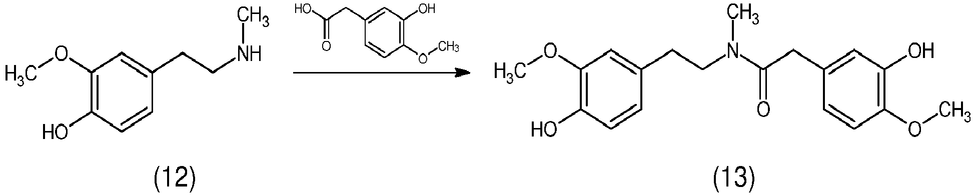 b) Bischler-Napieralski-Zyklisierung des tertiären Amids (13) zu 1,2-Dehydroreticulin (14): 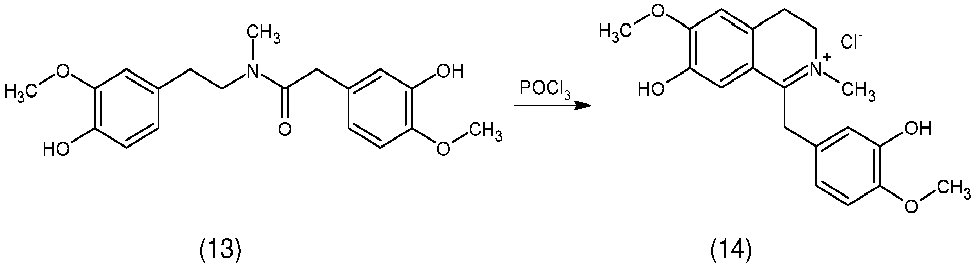 c) biokatalytische enantioselektive Hydrierung von 1,2-Dehydroreticulin (14) zu Reticulin (15) und zwar entweder
c1) unter Verwendung einer Imin-Reduktase zu (S)-Reticulin (S)-(15) oder
c2) unter Verwendung einer Dehydroreticulin-Reduktase zu (R)-Reticulin (R)-(15):

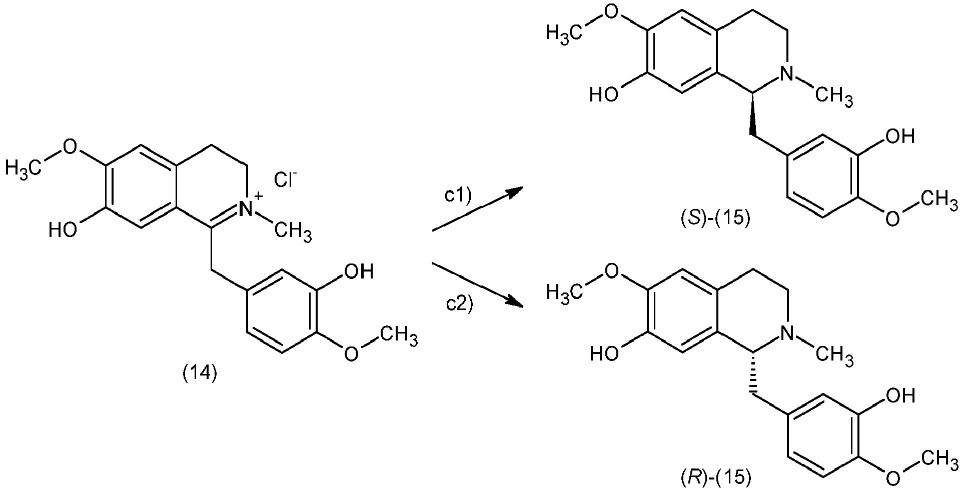

d) biokatalytische oxidative Phenol-Kupplung zwischen den beiden aromatischen Ringen des jeweiligen Reticulins und zwar entweder
d1) ortho-ortho-Kupplung von (S)-Reticulin in Gegenwart von Corytuberin-Synthase (CorSyn) zu Corytuberin (16) oder
d2) ortho-para-Kupplung von (R)-Reticulin in Gegenwart von Salutaridin-Synthase (SalSyn) zu Salutaridin (17):

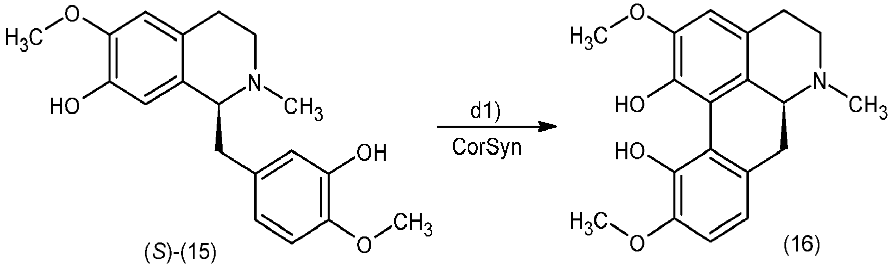

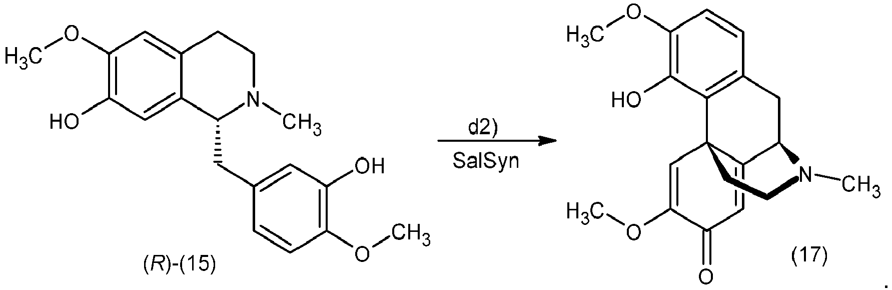

## Beschreibung

Die vorliegende Erfindung betrifft ein Verfahren zur Herstellung von Corytuberin und Salutaridin über Reticulin als Zwischenprodukt.

### STAND DER TECHNIK

Alkaloide sind Stickstoff-hältige, meist biologisch aktive sekundäre Stoffwechselprodukte. Eine Unterklasse davon sind Benzylisochinolin-Alkaloide (BIA), deren Struktur als Vorstufe für zahlreiche komplexere Verbindungen dient, aber auch die auf BIA-Strukturen basierenden Moleküle zeigen bereits biologische Aktivität. Alkaloide kommen in fast allen Klassen von Organismen vor, wo sie völlig unterschiedliche Rollen spielen können, beispielsweise zur Abwehr von Fressfeinden im Pflanzenreich. Diese Alkaloid-Strukturen spielen jedoch auch eine wichtige Rolle als API für die pharmazeutische und medizinische Industrie.

Eines der prominentesten Beispiele für Alkaloide ist Morphin, das der Mensch aufgrund der erstaunlichen Wirksamkeit des Opiumlatex seit mehr als fünftausend Jahren in verschiedensten Mischungen nutzt. Allerdings wurde erst zu Beginn des 19. Jahrhunderts Morphin als Hauptwirkstoff des Opiumextrakts identifiziert, was es zu einem wirksamen und starken Analgetikum macht. Und wenngleich die Struktur von Morphin bereits 1925 aufgeklärt wurde, ist es bis heute nur durch Isolierung aus Pflanzenmaterial in großen Mengen erhältlich, d. h. es gibt trotz zahlreicher Versuche zu seiner vollständig organisch-chemischen oder biochemischen/biotechnologischen (zumeist auf Metabolic Engineering beruhenden) Synthese weiterhin kein in industriellem Maßstab effizient durchführbares Syntheseverfahren für Morphin oder dessen natürliche Vorläufer, die Mono- bzw. Dimethylether-Derivate Codein und Thebain.

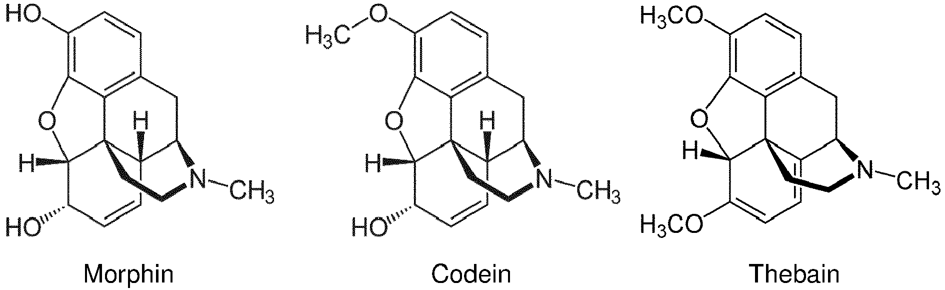

Der erste praktisch relevante Ansatz zur Synthese dieser Morphinan-Opiate stammt aus dem Jahr 1980 von Rice und kann als erster Meilenstein im Bereich der Totalsynthese angesehen werden (K. C. Rice, "Synthetic opium alkaloids and derivatives. A short total synthesis of (±)-dihydrothebainone, (±)-dihydrocodeinone, and (±)-nordihydrocodeinone as an approach to a practical synthesis of morphine, codeine, and congeners", J. Org. Chem. 45(15), 3135-3137 (1980), und WO 82/00144 A1). Dieser Ansatz umfasst unter anderem die 14-stufige Synthese des Morphin-Derivats Dihydrocodeinon in racemischer Form mit immerhin 29 % Gesamtausbeute, wonach allerdings fünf weitere Reaktionsschritte erforderlich sind, um die endgültige Morphinstruktur zu erhalten.

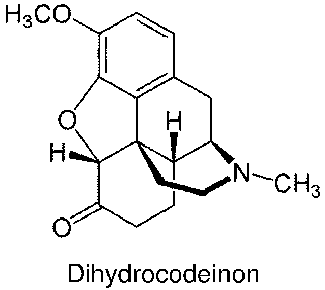

Seitdem wurden zahlreiche Totalsynthesen für die Herstellung von Morphinan-Verbindungen publiziert. Der nächste bemerkenswerte Erfolg wurde im Jahr 2009 von P. Magnus und seinen Mitarbeitern verzeichnet, die racemisches Codein, das ein direktes Morphin-Derivat darstellt, in 13 Syntheseschritten und mit 20 % Gesamtausbeute herstellen konnten (P. Magnus et al., "Concise Syntheses of (-)-Galanthamine and (±)-Codeine via Intramolecular Alkylation of a Phenol Derivative", J. Am. Chem. Soc. 131 (44), 16045-16047 (2009)).

Die erste relevante stereoselektive Syntheseroute eines Opiats wurde 2014 von Geffe und Opatz gezeigt (M. Geffe und T. Opatz, "Enantioselective Synthesis of (-)-Dihydro-codeine and Formal Synthesis of (-)-Thebaine, (-)-Codeine, and (-)-Morphine from a Deprotonated α-Aminonitrile", Org. Lett. 16(20), 5282-5285 (2014)). Ihnen gelang die Synthese von (-)-Dihydrocodein in 13 Syntheseschritten und mit 20 % Gesamtausbeute, ausgehend von dem einfachen Ausgangsmaterial Galussäuremethylester. Daraus konnten in weiteren Reaktionsschritten (-)-Codein und (-)-Morphin hergestellt werden.

Erst kürzlich zeigten Forscher der Arbeitsgruppen um Qin und Zhong einen Syntheseweg, der den Erhalt von (-)-Codein, (-)-Naloxon und (-)-Oxycodon in 12-15 Syntheseschritten und mit 16-34 % Gesamtausbeute ermöglichte, wobei allerdings von nicht kommerziell erhältlichen Ausgangsmaterialien ausgegangen wurde (H. He et al., "Concise total synthesis of opioids", Org. Chem. Front. 9(9), 2322-2327 (2022)).

Die letzte wichtige Publikation stammt von der Arbeitsgruppe um M. G. Banwell und P. Lan, die eine 7-stufige Synthese für die Herstellung von (-)-Codein publizierten (L. V. White et al., "Expeditious Access to Morphinans by Chemical Synthesis", Angew. Chem. Int. Ed. 61 (27), e202203186 (2022)). Diese stellt den bislang kürzesten Syntheseweg im Bereich der Totalsynthese von Opiatverbindungen dar.

Im Allgemeinen umfassen alle genannten Totalsynthesen eine hohe Anzahl an Syntheseschritten (ausgenommen die Publikation von L. V. White et al.) und den Einsatz zahlreicher Schutzgruppen. Darüber hinaus beinhalten fast alle genannten Synthesen mehrere Schritte, die mittels Übergangsmetallkomplexen katalysiert werden.

In den letzten Jahren wurden zunehmend biochemische und biotechnologische Verfahren zur Herstellung verschiedener Morphinan-Alkaloide untersucht. Beispielsweise offenbart die Arbeitsgruppe um Christina D. Smolke (Galanie et al., "Complete biosynthesis of opioids in yeast", Science 349(6252), 1095-100 (2015)) ein kompliziertes Pathwaydesign, auch Metabolic Engineering genannt, mittels dessen Thebain und Dihydrocodeinon unter Verwendung von 21 bzw. 23 Enzymen, beginnend mit einer Zuckerfermentierung mittels Backhefe (*Saccharamyces cerevisae*) hergestellt werden können. Zwar kann diese Arbeit durchaus als Meilenstein der biologischen Synthese angesehen werden, doch stellt sie nicht mehr als einen Proof of Concept dar, da die Ausbeuten nur zwischen 0,3 und 6,4 µg/l betrugen und damit um mehrere Zehnerpotenzen unter wirtschaftlich sinnvollen Werten lagen.

Ein Jahr später gelang es der Arbeitsgruppe um Hiromichi Minami (Nakagawa et al., "Total biosynthesis of opiates by stepwise fermentation using engineered Escherichia coli", Nature Communications 7(1), 10390 (2016)), die Ausbeuten an Thebain und Dihydrocodeinon um drei Zehnerpotenzen (auf 2,1 bzw. 0,36 mg/l) zu steigern, indem die Biosynthese statt mit Backhefe mit *Escherichia coli* durchgeführt wurde, und zwar in einem stufenweisen Kulturprozess unter Verwendung von vier getrennten *E. coli-*Kulturen, aus denen das jeweilige Produkt extrahiert und als Substrat für die nächste Fermentationsstufe eingesetzt wurde.

In den letztgenannten Arbeiten kamen hochkomplexe Enzyme, nämlich Cytochrome P450 (CYP) von höheren Pflanzen, in eukaryotischen, aber auch prokaryotischen Zellen zum Einsatz, wobei Letztere sogar höhere Ausbeuten generierten.

Darüber hinaus existieren mehrere Arbeiten, die sich mit der Herstellung eines essentiellen Zwischenprodukts der natürlichen Synthese dieser Alkaloide beschäftigen, nämlich des Benzylisochinolin-Alkaloids (S)-Reticulin:

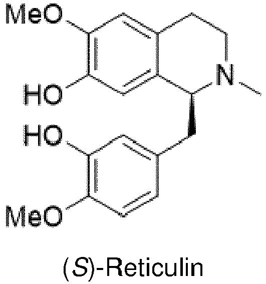

Während bei der Mehrzahl davon wiederum nur Ausbeuten im Bereich von wenigen Mikro- bis Milligramm pro Liter erzielt werden konnten, gelang vor zwei Jahren einer Arbeitsgruppe (Pyne et al., "A yeast platform for high-level synthesis of tetrahydroisoquinoline alkaloids", Nat. Commun. [Online] 2020, 3337) die Synthese dieser Verbindung unter Verwendung von *Saccharamyces cerevisae-Kulturen* in einer Ausbeute von 4,6 g/l, womit erstmals ein präparativ relevanter biotechnologischer Ansatz offenbart wurde.

(S)-Reticulin wird in der Natur in den Kapseln des Schlafmohns (*Papaver somniferum*) in einer zweistufigen enzymatischen Epimerisierung zuerst zu 1,2-Dehydroreticulin oxidiert, das anschließend zu (R)-Reticulin reduziert wird (das in der Folge zu Morphin oder Derivaten davon wie Codein und Thebain weiter umgesetzt wird):

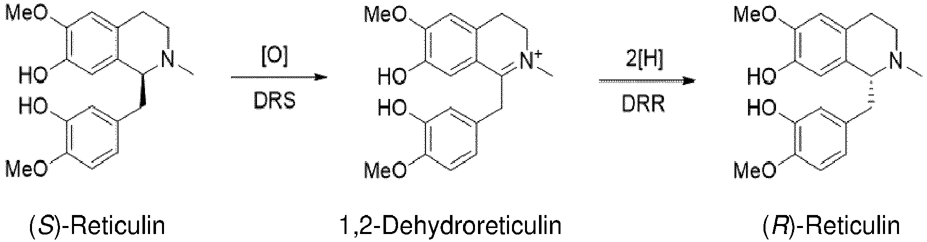

Wie die Arbeitsgruppe um Peter J. Facchini erst kürzlich festgestellt hat (Farrow et al., "Stereochemical inversion of (S)-reticuline by a cytochrome P450 fusion in opium poppy", Nat. Chem. Biol. 11(9), 728-32 (2015)), werden dabei beide Reaktionen von demselben Fusionsprotein, der (S)-Reticulin-Epimerase katalysiert, die im Englischen auch als STORR- ("(S)- to (R)-reticuline") Protein bekannt ist. Dieses besteht aus zwei Domänen: einer N-terminalen, die das Cytochrom P450-Enzym Dehydroreticulin-Synthase (DRS) umfasst, und einer C-terminalen Domäne, die die Aldo-Keto-Reduktase Dehydroreticulin-Reduktase (DRR) darstellt.

Weiters existieren einige wenige Publikationen zur Herstellung von Morphinan-Opiaten wie Morphin, Codein und Thebain unter Anwendung einer Kombination aus organisch-chemischer Synthese und enzymatischer Katalyse, bei denen letztere durchwegs als erstes zum Einsatz kam, um verschiedene Ausgangskomponenten für die späteren chemischen Synthesen der gewünschten Verbindungen herzustellen. Beispielsweise offenbart die Arbeitsgruppe um Tohru Fukuyama (Koizumi et al., "Total Synthesis of (-)-Morphine", Chem. Asian J. 5(10), 2192-2198 (2010)) eine 17-stufige Synthese zur Herstellung von Morphin in einer Gesamtausbeute von 5 %, wobei nur eine frühe Vorstufe durch enzymatische Katalyse unter Verwendung einer Lipase hergestellt wird. Und Bedard und Hudlicky beschreiben in "Chapter 2 - Enzymatic dihydroxylation of aromatic compounds: Nature's unique reaction and its impact on the synthesis of naturalproducts", Strategies and Tactics in Organic Synthesis, Harmata, M. (Hrsg), Academic Press, Bd. 15, S. 53-97 (2021), die enzymatische Dihydroxylierung von Phenethylbromid zur Herstellung einer Ausgangsverbindung für die anschließende chemische Synthese von Codein.

Zur Herstellung verschiedener Benzylisochinolin-Alkaloide als Vorstufen bei der Synthese von Morphinan-Strukturen wurden ebenfalls einige organisch-chemische Synthesewege publiziert, die üblicherweise eine Pictet-Spengler-Reaktion zur Herstellung von Heterozyklen durch Reaktion von mit Aromaten substituierten Ethylaminen mit Aldehyden und anschließende Zyklisierung unter Wasserabspaltung umfassen. Dazu sind in der Regel chirale Katalysatoren und zahlreiche Syntheseschritte erforderlich, und sowohl die Gesamtausbeuten als auch die Stereoselektivitäten sind üblicherweise gering (siehe Calcaterra et al., "The Pictet-Spengler Reaction Updates Its Habits", Molecules 25(2), 414 (2020)).

In der Natur wird diese Reaktion durch die Pictet-Spenglerase Norcoclaurin-Synthase (NCS) katalysiert, wobei das Benzylisochinolin-Alkaloid (S)-Norcoclaurin effizient und in optischer Reinheit erhältlich ist:

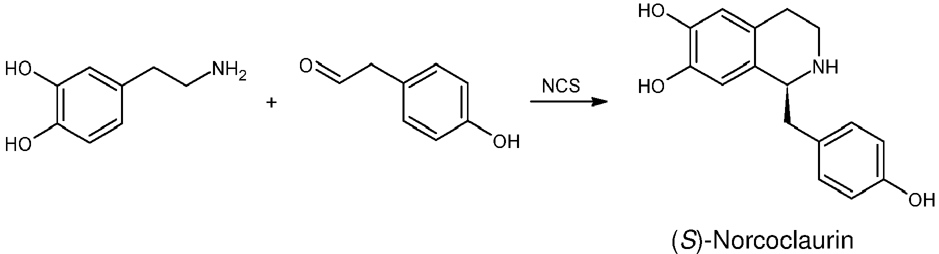

Erst kürzlich ist es der Arbeitsgruppe der vorliegenden Erfinder um Wolfgang Kroutil gelungen, diese NCS-katalysierte Biotransformation mittels einer In-vitro-Biokatalyse durchzuführen (siehe Cigan et al., "The rote of biocatalysis in the asymmetric synthesis of alkaloids - an update", RSC Advances 11(45), 28223-28270 (2021)). Allerdings mussten sie feststellen, dass NCS zwingend eine phenolische OH-Gruppe in meta-Stellung zum Ethylamin erfordert, wodurch sich diese Reaktion nicht zur Herstellung von Methoxy-Derivaten wie etwa Reticulin eignet.

Ziel der Erfindung war vor diesem Hintergrund die Entwicklung eines neuen Verfahrens, durch das mittels einer Kombination aus enzymatischer Katalyse und organischchemischer Synthese Benzylisochinolin-Alkaloide und andere essentielle Zwischenprodukte der Synthese verschiedener Alkaloide auf weniger aufwändige Weise und in guten Ausbeuten erhältlich sind.

### ZUSAMMENFASSUNG DER ERFINDUNG

Dieses Ziel erreicht die vorliegende Erfindung durch Bereitstellung eines Verfahrens zur Herstellung von Corytuberin und Salutaridin über das Benzylisochinolin-Alkaloid Reticulin als Zwischenprodukt, wobei das Verfahren die folgenden Reaktionsschritte umfasst:
a) Amidierung von 2-(4-Hydroxy-3-methoxyphenyl)-N-methylethanamin (12) mit 3-Hydroxy-4-methoxyphenylessigsäure zum N-[2-(4-Hydroxy-3-methoxyphenyl)ethyl-N-methyl-3-hydroxy-4-methoxyphenylessigsäureamid (13): 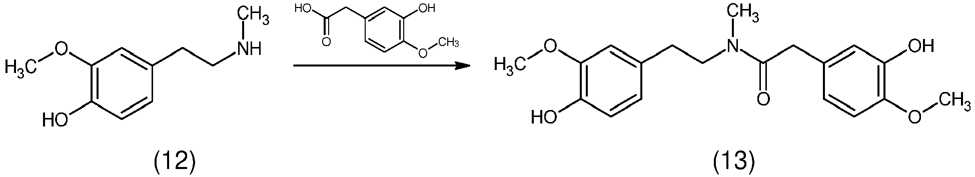
b) Bischler-Napieralski-Zyklisierung des tertiären Amids (13) zu 1,2-Dehydroreticulin (14): 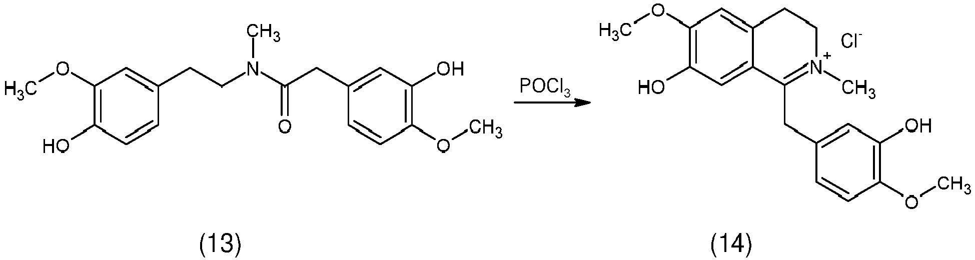
c) biokatalytische enantioselektive Hydrierung von 1,2-Dehydroreticulin (14) zu Reticulin (15) und zwar entweder
c1) unter Verwendung einer Imin-Reduktase zu (*S*)-Reticulin (*S*)-(15) oder
c2) unter Verwendung einer Dehydroreticulin-Reduktase zu (*R*)-Reticulin (*R*)-(15): 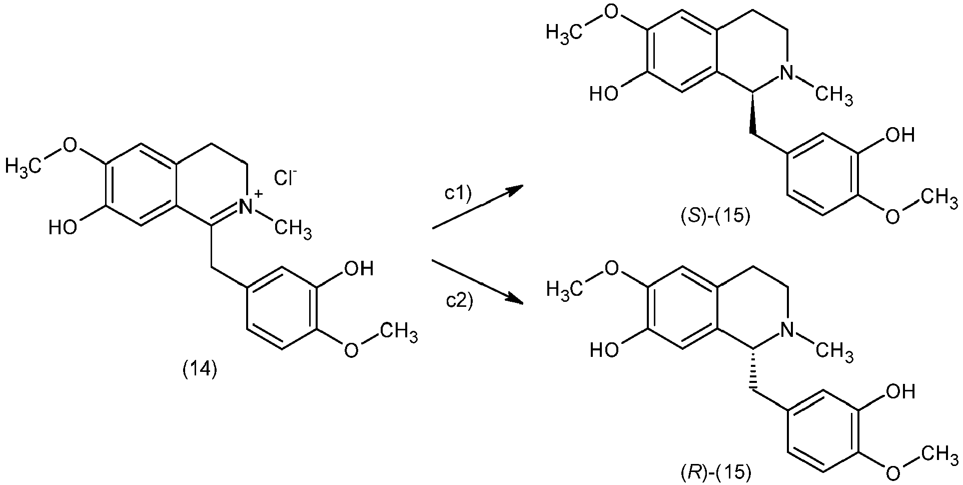
d) biokatalytische oxidative Phenol-Kupplung zwischen den beiden aromatischen Ringen des jeweiligen Reticulins und zwar entweder
d1) ortho-ortho-Kupplung von (S)-Reticulin in Gegenwart von Corytuberin-Synthase (CorSyn) zu Corytuberin (16) oder
d2) ortho-para-Kupplung von (R)-Reticulin in Gegenwart von Salutaridin-Synthase (SalSyn) zu Salutaridin (17):

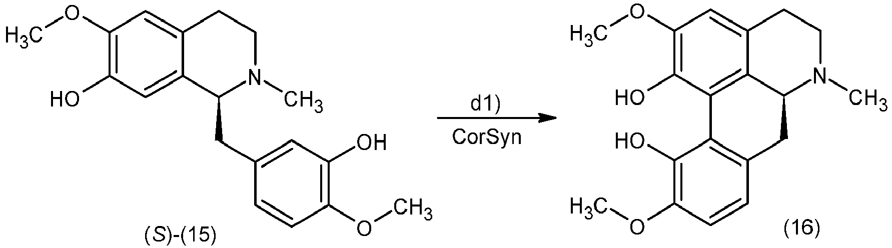

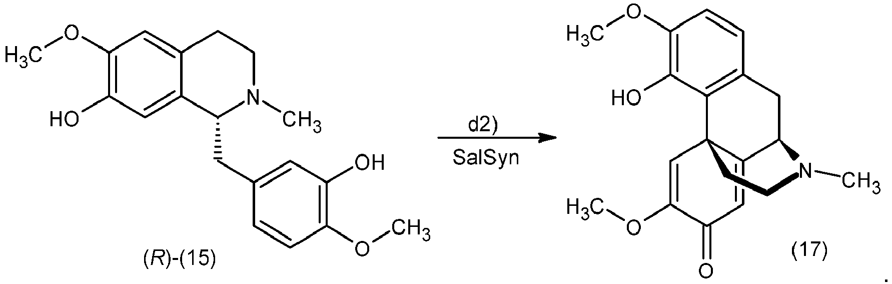

Auf diese Weise ist es gemäß vorliegender Erfindung möglich, mittels weniger Syntheseschritte zunächst das Benzylisochinolin-Alkaloid Reticulin - und zwar wahlweise in (S)- oder (R)-Konfiguration - und daraus in nur einer Stufe entweder Corytuberin oder Salutaridin herzustellen, wovon das erste bereits ein Aporphin-Alkaloid ist und das zweite eine Vorstufe für die Synthese von Morphinan-Alkaloiden darstellt. So kann beispielsweise aus Corytuberin (16) durch Zyklisierung das Aporphin-Alkaloid Bulbocapnin gebildet werden:

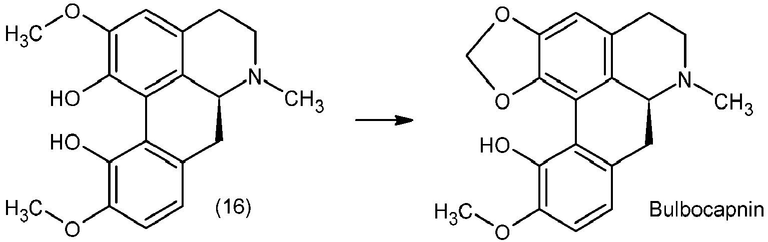

Und Salutaridin kann zunächst (z.B. mittels einer Oxidoreduktase) zu Salutaridinol reduziert werden, das in der Folge (z.B. mittels einer O-Acetyltransferase und Acetyl-CoA) acetyliert und anschließend unter Abspaltung des Acetats zu Thebain zyklisiert werden kann:

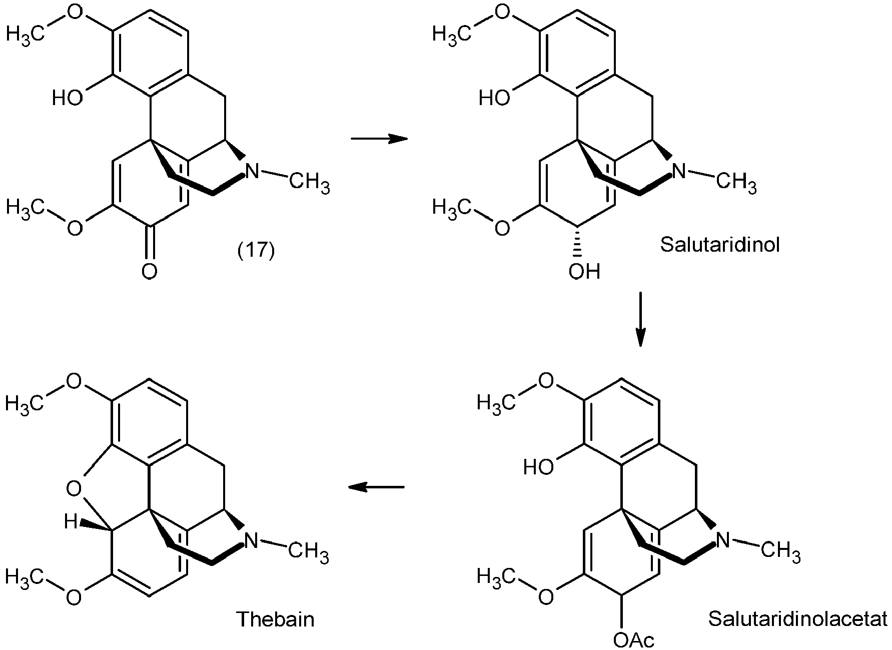

Darüber hinaus sind bereits die anfänglichen organisch-chemischen Reaktionen in hohen Ausbeuten ausführbar, und die beiden nachfolgenden Biokatalysen ergeben die gewünschten Produkte jeweils in praktisch quantitativer Ausbeute und mit nahezu 100%iger Enantiomerenreinheit (> 99 % ee).

Dazu kommt, dass das Ausgangsprodukt 2-(4-Hydroxy-3-methoxyphenyl)-N-methyl-ethanamin (12) durch einen direkten Methylierungsschritt aus im Handel erhältlichem 2-(4-Hydroxy-3-methoxyphenyl)ethanamin zugänglich ist, obwohl es in bevorzugten Ausführungsformen der Erfindung stattdessen in drei einfachen Syntheseschritten aus einem erheblich kostengünstigeren Ausgangsprodukt hergestellt wird, wie dies später näher ausgeführt wird.

Die Amidierung in Schritt a) könnte zwar auch in allgemein bekannter Weise unter Verwendung des Säurechlorids von 3-Hydroxy-4-methoxyphenylessigsäure durchgeführt werden, wozu allerdings ein zusätzlicher Schritt zu dessen Synthese aus der im Handel erhältlichen freien Säure erforderlich wäre. Daher wird das Amid (13) gemäß vorliegender Erfindung wie oben angegeben aus der freien Säure und dem sekundären Amin (12) unter Wasserabspaltung hergestellt. Dies kann beispielsweise durch starkes Erhitzen der Reaktanden erfolgen, wird vorzugsweise aber unter Verwendung eines Carbodiimids zur Aktivierung der Säure durchgeführt, wobei als Carbodiimid besonders bevorzugt 1-Ethyl-3-(3-dimethylaminopropyl)carbodiimid (EDC) eingesetzt wird, da der als Nebenprodukt entstehende Harnstoff leicht abtrennbar ist. Insbesondere wird dabei Hydroxyiminocyanessigsäureethylester (Oxyma) als Additiv zugesetzt, das die Entstehung unerwünschter Nebenprodukte, z.B. des unreaktiven N-Acyl-Harnstoffs, verhindert und somit die Ausbeuten des gewünschten Amids erhöht.

Die Bischler-Napieralski-Zyklisierung in Schritt b) wird in einem aprotischen polaren Lösungsmittel, vorzugsweise in Acetonitril, noch bevorzugter unter Rückfluss, durchgeführt und liefert 1 ,2-Dehydroreticulin (14) in hoher Ausbeute.

Dessen biokatalytische enantioselektive Hydrierung in Schritt c1) erfolgt mittels einer Imin-Reduktase, wofür eine Vielzahl von Enzymen zur Verfügung steht. In bevorzugten Ausführungsformen der Erfindung wird eine der insgesamt 88 von Zhu et al. in "Enantioselective Synthesis of 1-Aryl-Substituted Tetrahydroisoquinolines Employing Imine Reductase", ACS Catalysis 7(10), 7003-7007 (2017)), für die Reduktion einiger 1-Aryldihydroisochinoline beschriebenen Imin-Reduktasen eingesetzt. Noch bevorzugter wird gemäß vorliegender Erfindung jedoch die von derselben Arbeitsgruppe in Yang et al., "Biosynthesis of plant tetrahydroisoquinoline alkaloids through an imine reductase route", Chemical Science 11(2), 364-371 (2020)), offenbarte Imin-Reduktase IR45 F190M-W191F eingesetzt, da sich diese bei der Reduktion verschiedener 1-Phenyldihydroisochinoline, aber auch von mehreren 1-Benzyldihydroisochinolinen bewährt hat, die eine ähnliche Struktur aufweisen wie 1,2-Dehydroreticulin.

Dabei wird vorzugsweise ein von mit dem entsprechenden Gen transformierten Zellen heterolog exprimiertes rekombinantes Enzym eingesetzt, wobei noch bevorzugter ein von *E*. c*oli-*Zellen, insbesondere von kompetenten BL21(DE3)-Zellen, heterolog exprimiertes rekombinantes Enzym in Form der lyophilisierten Zellen oder eines zellfreien Extrakts davon eingesetzt wird. Die besten Ergebnisse wurden mit einem zellfreien Extrakt von in Terrific-Broth- (TB-) Medium kultivierten transformierten BL21(DE3)-Zellen erzielt.

Die sonstigen Bedingungen der Hydrierung in Schritt c1) sind nicht speziell eingeschränkt, wobei vorzugsweise jedoch eine oder mehrere oder alle der folgenden Bedingungen i) bis v) eingestellt werden:
i) 0,1 M KPi-Puffer wird als Lösungsmittel eingesetzt;
ii) die Dehydroreticulin-Konzentration beträgt 5-30 mM, noch bevorzugter 10-15 mM, insbesondere 10 mM;
iii) der pH beträgt 7-8,5, noch bevorzugter 7,5-8,0;
iv) NADPH/NADP⁺ wird als Cofaktor, insbesondere in einer Konzentration von 1 mM, eingesetzt;
v) Glucose-Dehydrogenase wird als Co-Enzym, insbesondere in einer Konzentration von 2 mg/ml, und D-Glucose als Co-Substrat, insbesondere in einer Konzentration von 9 mg/ml, eingesetzt.

Die biokatalytische enantioselektive Hydrierung von 1 ,2-Dehydroreticulin zu (R)-Reticulin in Schritt c2) unter Dehydroreticulin-Reduktase-Katalyse wird vorzugsweise unter Verwendung der Dehydroreticulin-Reduktase von *Papaver somniferum* (PsDRR) durchgeführt, die Teil der (S)-Reticulin-Epimerase in Schlafmohn-Kapseln ist und diese Hydrierungsreaktion auch in der Natur katalysiert. Auch in diesem Fall wird besonders bevorzugt ein von mit dem entsprechenden Gen transformierten Zellen heterolog exprimiertes rekombinantes Enzym eingesetzt, noch bevorzugter ein von *E. coli-Zel*len, insbesondere ein von kompetenten Arctic Express (DE3)-Zellen, exprimiertes Enzym in Form der lyophilisierten Zellen oder eines zellfreien Extrakts davon. Die besten Ergebnisse wurden mit in Lysogeny-Broth- (LB-) Medium kultivierten transformierten Arctic Express (DE3)-Zellen, genauer gesagt mit den lyophilisierten ganzen Zellen erzielt.

Die sonstigen Bedingungen der Hydrierung in Schritt c2) sind nicht speziell eingeschränkt, wobei vorzugsweise jedoch eine oder mehrere oder alle der folgenden Bedingungen i) bis vi) eingestellt werden:
i) 0,1 M Tris-HCl-Puffer oder Glycin-NaOH-Puffer wird als Lösungsmittel eingesetzt;
ii) 5-10 Vol.-%, insbesondere 10 Vol.-%, DMSO, werden als Co-Lösungsmittel eingesetzt;
iii) die Dehydroreticulin-Konzentration beträgt 10-30 mM, insbesondere 10-20 mM;
iv) der pH beträgt 8-8,5 (Tris-HCl) bzw. 9-9,5 (Glycin-NaOH);
v) NADPH/NADP⁺ wird als Cofaktor, insbesondere in einer Konzentration von 1 mM, eingesetzt;
vi) Glucose-Dehydrogenase wird als Co-Enzym, insbesondere in einer Konzentration von 2 mg/ml, und D-Glucose als Co-Substrat, insbesondere in einer Konzentration von 9 mg/ml, eingesetzt.

In weiteren bevorzugten Ausführungsformen der vorliegenden Erfindung wird die biokatalytische oxidative Phenol-Kupplung zwischen den beiden aromatischen Ringen des (S)-Reticulins in Schritt d1) unter Verwendung einer Corytuberin-Synthase, vorzugsweise der Corytuberin-Synthase von *Coptis japonica* (CjCorSyn) oder einer modifizierten Version davon, zusammen mit der Cytochrom P450-Reduktase 2 von *Arabidopsis thaliana (AtR2)* als Redoxpartner durchgeführt, um die Effizienz des Katalysesystems zu optimieren. Besonders bevorzugt werden die Corytuberin-Synthase und die AtR2 als von mit den entsprechenden Genen transformierten Zellen heterolog exprimierte rekombinante Enzyme eingesetzt, noch bevorzugter die von *E.* coli-Zellen, insbesondere wiederum von kompetenten BL21 (DE3)-Zellen, exprimierten Enzyme in Form der ganzen Zellen oder eines zellfreien Extrakts davon. Die besten Ergebnisse wurden mit in TB-Medium kultivierten transformierten BL21 (DE3)-Zellen, genauer gesagt mit den resuspendierten ganzen Zellen erzielt.

Die sonstigen Bedingungen der ortho-ortho-Phenol-Kupplung in Schritt d1) sind nicht speziell eingeschränkt, wobei vorzugsweise jedoch eine oder mehrere oder alle der folgenden Bedingungen i) bis vii) eingestellt werden:
i) 0,1 M KPi-Puffer wird als Lösungsmittel eingesetzt;
ii) die Enzyme werden in Form von resuspendierten ganzen Zellen eingesetzt;
iii) 0,1-5 Vol.-%, insbesondere 0,2-1 Vol.-%, DMSO werden als Co-Lösungsmittel eingesetzt;
iv) die Reticulin-Konzentration beträgt 0,1-5 mM;
v) der pH beträgt 7,5-8,0;
vi) NADPH/NADP⁺ wird als Cofaktor, insbesondere in einer Konzentration von 0,05 mM, eingesetzt;
vii) ein Glucose-Dehydrogenase-Präparat wird als Co-Enzym, insbesondere in einer Konzentration von 2 mg/ml, und D-Glucose als Co-Substrat, insbesondere in einer Konzentration von 50 mM, eingesetzt.

Gemäß einer weiteren bevorzugten Ausführungsform wird die biokatalytische oxidative Phenol-Kupplung zwischen den beiden aromatischen Ringen des (R)-Reticulins in Schritt d2) unter Verwendung der Salutaridin-Synthase von *Papaver somniferum* (PsSalSyn), einer chimären Version davon, die am N-terminalen Ende eine Peptidsequenz eines anderen Cytochrom-P450-Enzyms, nämlich von Cheilanthifolin-Synthase CYP719A5 umfasst (CFS_*Ps*SalSyn), oder einem Konstrukt, bei dem der N-terminale Membranteil durch eine hydrophile Peptidsequenz ausgetauscht wurde (NswapKK_SalSyn), durchgeführt. Noch bevorzugter wird die Salutaridin-Synthase zusammen mit der Cytochrom P450-Reduktase 2 von *Arabidopsis thaliana* (*AtR2*) als Redoxpartner eingesetzt. Besonders bevorzugt werden die Salutaridin-Synthase und die AtR2 als von mit den entsprechenden Genen transformierten Zellen heterolog exprimierte rekombinante Enzyme eingesetzt, noch bevorzugter die von *E.* coli-Zellen, insbesondere erneut die von kompetenten BL21 (DE3)-Zellen, exprimierten Enzyme in Form der lyophilisierten Zellen oder eines zellfreien Extrakts davon. Die besten Ergebnisse wurden mit in TB-Medium kultivierten transformierten BL21 (DE3)-Zellen, genauer gesagt mit den resuspendierten ganzen Zellen erzielt.

Die sonstigen Bedingungen der ortho-para-Phenol-Kupplung in Schritt d2) sind nicht speziell eingeschränkt, wobei vorzugsweise jedoch eine oder mehrere oder alle der folgenden Bedingungen i) bis vii) eingestellt werden:
i) 0,1 M KPi-Puffer wird als Lösungsmittel eingesetzt;
ii) die Enzyme werden in Form von resuspendierten ganzen Zellen eingesetzt;
iii) 0,1-5 Vol.-%, insbesondere 0,2-2 Vol.-%, DMSO werden als Co-Lösungsmittel eingesetzt;
iv) die Reticulin-Konzentration beträgt 0,1-0,5 mM;
v) der pH beträgt 7,5-8,0;
vi) NADPH/NADP⁺ wird als Cofaktor, insbesondere in einer Konzentration von 0,05 mM, eingesetzt;
vii) ein Glucose-Dehydrogenase-Präparat wird als Co-Enzym, insbesondere in einer Konzentration von 2 mg/ml, und D-Glucose als Co-Substrat, insbesondere in einer Konzentration von 50 mM, eingesetzt.

Wie zuvor erwähnt wird in besonders bevorzugten Ausführungsformen des Verfahrens der vorliegenden Erfindung das in Schritt a) eingesetzte Ausgangsprodukt 2-(4-Hydro-xy-3-methoxyphenyl)-N-methylethanamin (12) zuvor ausgehend von 4-Allyl-2-methoxyphenol (Eugenol) durch die folgenden Reaktionsschritte hergestellt:
I) Ozonolyse von 4-Allyl-2-methoxyphenol (9) zu 4-Hydroxy-3-methoxyphenyl-acetaldehyd (Homovanillin) (10): 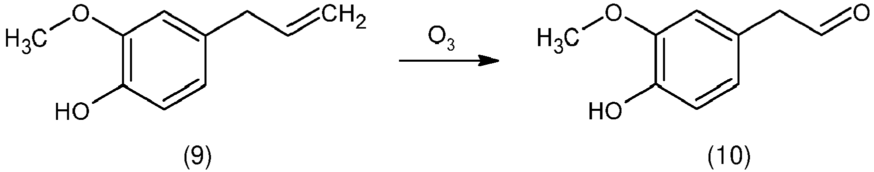
II) reduktive Aminierung des Aldehyds (10) mit N-Benzylmethanamin zum Benzyl-geschützten Zwischenprodukt N-Benzyl-2-(4-hydroxy-3-methoxyphenyl)-N-methylethanamin (11): 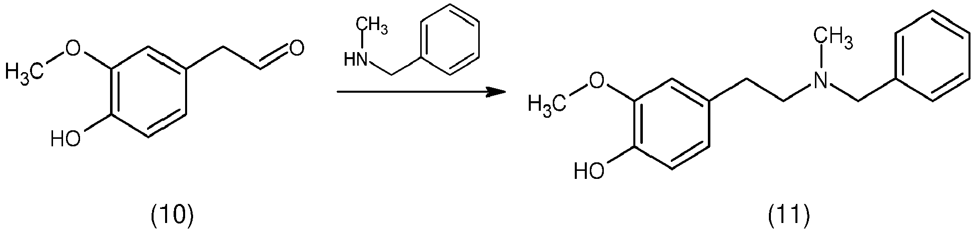
III) Hydrogenolyse zur Entfernung der Benzyl-Schutzgruppe, um das gewünschte Amin (12) zu erhalten:

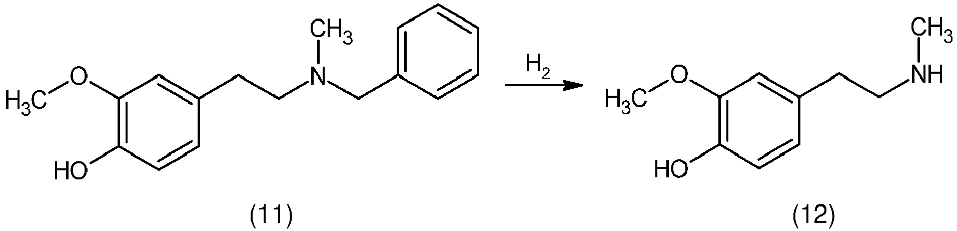

Das hier als Ausgangsprodukt eingesetzte Eugenol (9) ist im Handel zu einem erheblich niedrigeren Preis erhältlich, und die Produkte der Reaktionsschritte I) bis III) sind alle in sehr guten Ausbeuten herstellbar - speziell nach einer Optimierung der Reaktionen auf die nachstehende Weise.

Vorzugsweise wird die Ozonolyse von Eugenol (9) in Schritt I) in organischer Lösung durch Einleiten eines Gemischs aus O₃/O₂ bei einer Temperatur < 0 °C, noch bevorzugter bei -15 °C, und anschließendes Quenchen des Ozonids mit einem Reduktionsmittel, noch bevorzugter mit Dimethylsulfid, durchgeführt; und/oder
die reduktive Aminierung des Aldehyds (10) in Schritt II) wird in organischer Lösung in Gegenwart eines Reduktionsmittels, noch bevorzugter eines Borhydrids, insbesondere von NaBH(OAc)₃, unter Eisbadkühlung durchgeführt;
   und/oder
die Hydrogenolyse in Schritt III) wird in alkoholischer Lösung in Gegenwart eines Palladium-Katalysators, noch bevorzugter von Pd/C, und von HCl bei Raumtemperatur durchgeführt;
wobei die sonstigen Bedingungen nicht speziell eingeschränkt sind und in jedem Einzelfall gesondert optimiert werden können.

Die reduktive Aminierung von Homovanillin (10) mit N-Benzylmethanamin zum Benzyl-geschützten Zwischenprodukt (11) ist deswegen zu bevorzugen, weil bei der direkten Reaktion mit Methylamin zum Amin (12) verschiedene Nebenreaktionen auftreten können: Bei der organisch-chemischen Synthese kommt es leicht zu einer Überalkylierung, und bei enzymatischer Katalyse in basischer wässriger Lösung neigt der Aldehyd (10) zu spontaner Dimerisierung.

In einer besonders bevorzugten Ausführungsform der Erfindung werden zudem die Schritte I) und II) als Eintopfreaktion durchgeführt, wodurch sich die Isolierung von Homovanillin (10) erübrigt, was die Gesamtausbeute erhöht.

### BEISPIELE

Die vorliegende Erfindung wird nachstehend anhand von repräsentativen, nicht einschränkenden Beispielen näher beschrieben, die nur zu Illustrationszwecken angeführt werden.

### Materialien und Verfahren

Alle Chemikalien wurden von Sigma Aldrich, Acros Organics, Alfa Aesar oder ABCR erstanden und ohne weitere Reinigung eingesetzt. Alle Lösungsmittel wurden von Roth bezogen. Mit "abs." gekennzeichnete Lösungsmittel wurden durch Absolutierung getrocknet. Wasser in Laborqualität wurde mittels hauseigener Ausrüstung (Entsalzungsapparat) hergestellt. Genstränge wurden von Thermo Fisher Scientific bei Invitrogen und Plasmide, die Gene enthielten, entweder von der BioCat GmbH oder von General Biosystems bezogen. Sofern nicht anders angegeben wurde die jeweils angegebene Ausrüstung unter Standardbedingungen eingesetzt und haben alle Begriffe, chemischen Bezeichnungen und Abkürzungen die übliche, durchschnittlichen Fachleuten wohlbekannte Bedeutung.

Die Reaktionen wurden mittels Dünnschichtchromatographie (DC) überwacht, wobei als stationäre Phase Kieselgel/Aluminium-Platten von Merck (Kieselgel 60 F₂₅₄ Aluminum DC-Platten, 20x20 cm) eingesetzt wurden. Die Detektion erfolgte mit UV-Licht (254 nm) oder Färbung mit "CAM Blue"- (Ceric Ammonium Molybdate-) Lösung, die durch Lösen von 2,5 g Ammoniummolybdat-tetrahydrat und 1 g Ammoniumcer(IV)-sulfat-dihydrate in 90 ml dest. H₂O unter Rühren und anschließende Zugabe von 10 ml konz. H₂SO₄ erhalten wurde. Alle GC-MS-Messungen wurden mit einem Agilent 7890A GC-System, ausgestattet mit einem Agilent 5975C massenselektiven Detektor (Elektronenstoß, 70 eV) und einer HP-5-MS-Säule (30 m x 0,25 mm x 0,25 µm Film) unter Verwendung von He als Trägergas mit einer Durchflussrate von 0,55 ml/min durchgeführt. GC-FID-Analysen wurden mit einem Agilent 7890A GC-System und einer HP-5-Säule (30 m x 0,32 mm x 0,25 µm Film) unter Verwendung von H₂ als Trägergas mit einer Durchflussrate von 0,55 ml/min durchgeführt. Massenspektren mit niedriger Auflösung wurden auf einem Agilent Technologies 6120 Quadrupole LC/MS-Detektor in Kombination mit einem Agilent Technologies 1260 Infinity HPLC-System aufgenommen, das mit einer Kinetex 2,6 µ C-18 100A-Säule (50 x 4,6 mm, 2,6 Mikrometer) ausgestattet war. Als Laufmittel wurde Wasser/ Acetonitril (+ 0,1 Vol.-% Ameisensäure) eingesetzt. HPLC-UV-Analysen wurden auf einem Shimadzu HPLC-System, das mit einer Luna 5 µ C-18 100A-Säule (250 x 4,6 mm, 5 µm) [DGU-20A (Entgaser), LC-20A (Pumpe), SIL-20A (Autosampler), CTO-20AC (Säulenofen), SPD-M20A (Detektor), CBM-20AC (Controller)] ausgestattet war, mit unterschiedlichen Eluentensystemen durchgeführt.

### Beispiel 1

### Herstellung von 4-Hydroxy-3-methoxyphenylacetaldehyd (Homovanillin) (10) aus 4-Allyl-2-methoxyphenol (Eugenol) (9)

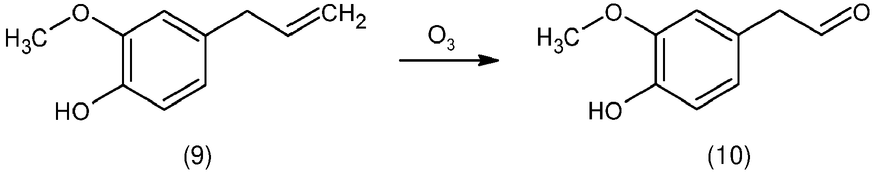

Ein Dreihalsrundkolben (500 ml) mit einem Magnetrührstäbchen wurde mit Eugenol (9) (5,00 g, 30,5 mmol, 1 Äqu.), gefolgt von CH₂Cl₂ (250 ml) beschickt. Die erhaltene gelbe Lösung wurde mit einem Kryostaten in einem EtOH-Bad auf -15 °C gekühlt, wonach durch eine Sinterglasfritte ein O₃/O₂-Gemisch 6 h lang durch die Lösung geleitet wurde. Nach vollständigem Verbrauch des Ausgangsmaterials (DC, EtOAc/Cyclohexan = 3:1, Rf = 0,54) wurde 10 min lang Stickstoff durch die Lösung geleitet, um den Ozon-Überschuss zu entfernen. Das reaktive Zwischenprodukt wurde durch Zugabe von Me₂S (11,2 ml, 153 mmol, 5 Äqu.) bei -15 °C gequencht und nach 15 min wurde das Gemisch auf Raumtemperatur erwärmen gelassen. Nach vollständigem Umsatz des Hydroperoxyacetals (DC, Cyclohexan/EtOAc = 1:1, Rf = 0,36) wurde das Gemisch im Vakuum eingeengt. Das Produkt wurde durch Säulenchromatographie gereinigt (Säule: 30 x 6 cm, Cyclohexan/EtOAc = 3:1 (Vol./Vol.). Ausbeute: 3,77 g (22,7 mmol, 74 % d. Th.) blassgelber Feststoff.

### Beispiel 2

### Herstellung von N-Benzyl-2-(4-hydroxy-3-methoxyphenyl)-N-methylethanamin (11) aus 3-Hydroxy-4-methoxyphenylacetaldehyd (10)

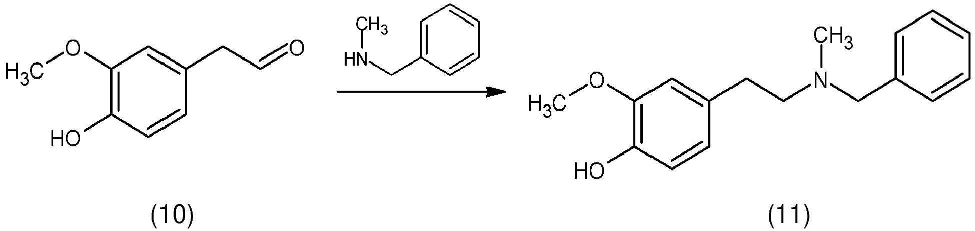

Ein trockener 500-ml-Rundkolben mit Magnetrührstäbchen wurde mit 4,00 g (24,1 mmol, 1 Äqu.) 2-(4-Hydroxy-3-methoxyphenyl)acetaldehyd (10) und 240 ml CH₂Cl₂ beschickt. Die blassgelbe Lösung wurde mit einem Eisbad auf 0 °C gekühlt, wonach 4,80 ml (36,1 mmol, 1,5 Äqu.) N-Methyl-1-phenylmethanamin langsam zugegeben wurden. 10,4 g (48,1 mmol, 2 Äqu.) NaBH(OAc)₃ wurden portionsweise über einen Zeitraum von 3 min zugegeben, der Kolben mit einem Septum verschlossen und das Gemisch im auftauenden Eisbad rühren gelassen. Nach 150 min wurde mittels DC unvollständiger Umsatz des Aldehyds nachgewiesen, weswegen eine weitere Portion NaBH(OAc)₃ (2,60 g, 0,5 Äq.) bei Raumtemperatur zugesetzt wurde. Vollständiger Umsatz des Ausgangsmaterials wurde nach insgesamt 18 h festgestellt, und das Reaktionsgemisch wurde durch Zugabe von gesättigter wässriger NaHCOs-Lösung und festem NaHCOs gequencht. Die Lösungsmittel wurden am Rotationsverdampfer entfernt (Badtemperatur: 50 °C) und das Produkt durch zweimalige Säulenchromatographie (1. Säule: 17 x 4,8 cm, CH₂Cl₂/Aceton = 1:1; 2. Säule: 16 x 4,8 cm, CH₂Cl₂/ Aceton = 2:1) gereinigt. Ausbeute: 5,15 g (19,0 mmol, 79 % d. Th.) hellbrauner Feststoff.

### Beispiel 3

### Herstellung von N-Benzyl-2-(4-hydroxy-3-methoxyphenyl)-N-methylethanamin (11) aus 4-Allyl-2-methoxyphenol (9) im Eintopfverfahren

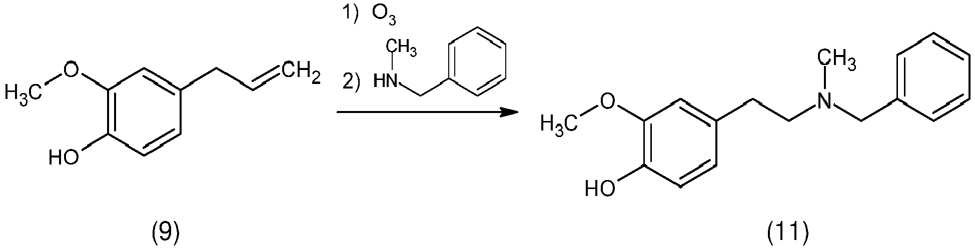

Zunächst wurde Homovanillin (10) wie in Beispiel 1 angegeben hergestellt, wobei nach vollständigem Verbrauch des Ausgangsmaterials (DC, EtOAc/Cyclohexan = 3:1, Rf = 0,54) 10 min lang Stickstoff durch die Lösung geleitet wurde, um den Ozon-Überschuss zu entfernen. Das reaktive Zwischenprodukt wurde durch Zugabe von Me₂S (11,2 ml, 153 mmol, 5 Äqu.) bei -15 °C gequencht, und nach 15 min wurde das Gemisch auf Raumtemperatur erwärmen und über Nacht rühren gelassen. Die dabei erhaltene blassgelbe Lösung wurde mit einem Eisbad auf 0 °C gekühlt, wonach die Reaktion des Zwischenprodukts (10) zur Titelverbindung (11) und deren Aufarbeitung und Reinigung wie in Beispiel 2 mit 6,10 ml (45,9 mmol, 1,5 Äqu.) N-Methyl-1-phenyl-methanamin und 25,7 g (122,4 mmol, 4 Äqu.) NaBH(OAc)₃ wie in Beispiel 2 erfolgten. Ausbeute: 6,6 g (24,5 mmol, 80 % d. Th.) hellbrauner Feststoff.

### Beispiel 4

### Herstellung von 2-(4-Hydroxy-3-methoxyphenyl)-N-methylethanamin (12)

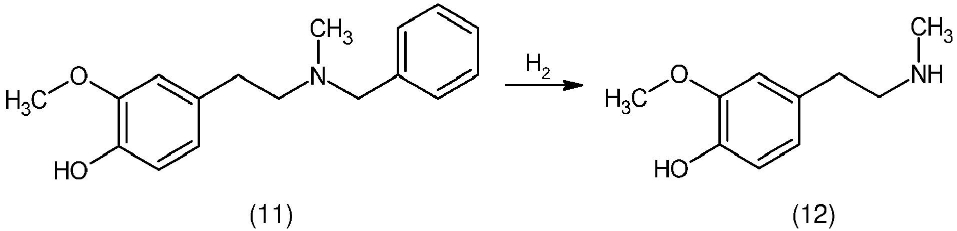

Ein 1000-ml-Rundkolben mit einem Magnetrührstäbchen wurde mit 4,35 g (16,0 mmol, 1 Äqu.) N-Benzyl-2-(4-hydroxy-3-methoxyphenyl)-N-methylethanamin (11), 160 ml 2-Propanol, 2,7 ml (32,0 mmol, 2 Äqu.) konz. wässrige HCl (12 M) und 218 mg Pd/C (10 %) beschickt. Das Gemisch wurde unter Wasserstoffatmosphäre bei 22 °C heftig gerührt, bis nach 3 d vollständiger Umsatz des Ausgangsmaterials festgestellt wurde (DC). Das Gemisch wurde durch ein Celite-Kissen (6 cm) filtriert und das Kissen wurde nacheinander mit 2-Propanol (500 ml), 2-Propanol/H₂O (1:1, 300 ml) und MeOH (400 ml) gespült. Die Lösungsmittel wurden am Rotationsverdampfer entfernt und das Produkt im Vakuum getrocknet, wobei (12) als Hydrochlorid erhalten wurde. Ausbeute: 3,13 g (14,4 mmol, 90 % d. Th.) brauner Feststoff.

### Beispiel 5

### Herstellung von N-(4-Hydroxy-3-methoxyphenethyl)-2-(3-hydroxy-4-methoxyphenyl)-N-methylacetamid (13)

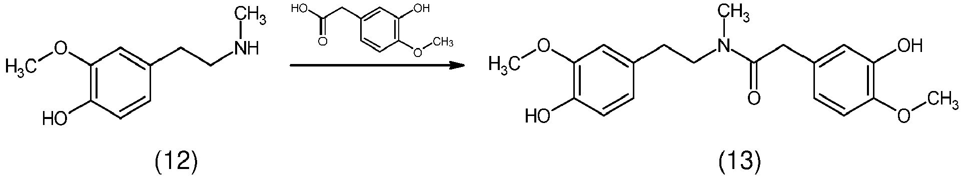

Ein trockener 250-ml-Rundkolben mit einem Magnetrührstäbchen wurde mit 560 mg (2,57 mmol, 1,05 Äqu.) des in Beispiel 4 erhaltenen Hydrochlorids von (12), 20 ml DMF und 346 mg (2.44 mmol, 1 Äqu.) Oxyma Pure beschickt. Die Lösung wurde mit einem Eisbad auf 0 °C gekühlt, wonach 0,34 ml (2,44 mmol, 1 Äqu.) EtsN, gefolgt von 520 mg (2,69 mmol, 1,1 Äqu.) 1-Ethyl-3-(3-dimethylaminopropyl)carbodiimid-hydrochlorid (EDC.HCl) zugesetzt wurden. Zu der heftig gerührten Lösung wurden 463 mg (2,44 mmol, 1 Äqu.) 2-(3-Hydroxy-4-methoxyphenyl)essigsäure in 4 ml DMF über einen Zeitraum von 3 h mit einer Spritzenpumpe zugetropft. Vollständiger Umsatz des Ausgangsmaterials wurde nach 18,5 h (DC) nachgewiesen, und das Lösungsmittel wurde am Rotationsverdampfer entfernt. Der zurückgebliebene Feststoff wurde in 70 ml EtOAc aufgenommen und mit wässriger 1 M HCl-Lösung gewaschen (3 x 50 ml). Die vereinigte wässrige Waschlösung wurde mit NaHCOs neutralisiert und erneut mit EtOAc extrahiert (3 x 70 ml). Die organischen Phasen wurden mit den vorangegangenen organischen Phasen vereinigt und am Rotationsverdampfer eingeengt. Das Produkt wurde durch Säulenchromatographie gereinigt (Säule: 26 x 3,3 cm, EtOAc/Cyclohexan = 2:1, 4:1 (Vol./Vol.). Das Produkt wurde im Ölpumpenvakuum getrocknet (Achtung: starkes Schäumen!). Ausbeute: 807 mg (2,34 mmol, 96 % d. Th.) farbloser, glasartiger Feststoff.

### Beispiel 6

### Herstellung von 1,2-Dehydroreticulin (14):

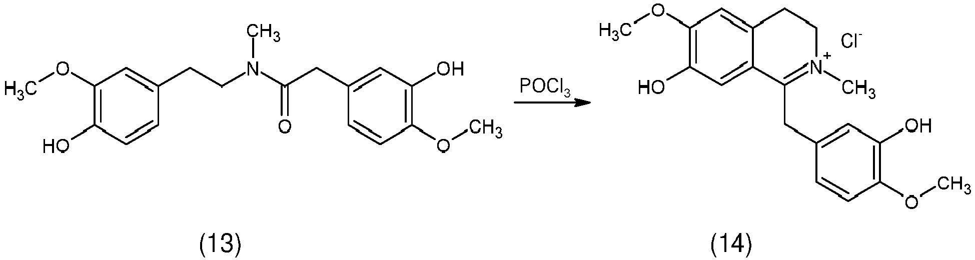

Ein 250-ml-Dreihalsrundkolben mit Magnetrührer, Luftkühler, Druckausgleich und Gasventil wurde 45 min lang mit Argon gespült, wonach im Ar-Gegenstrom 1,00 g (2,90 mmol, 1 Äqu.) N-(4-Hydroxy-3-methoxyphenethyl)-2-(3-hydroxy-4-methoxyphenyl)-N-methylacetamid (13) in 30 ml trockenem MeCN, gefolgt von 824 µl (8,69 mmol, 3 Äqu.) POCl₃ zugesetzt wurden. Die farblose Lösung wurde rückflusserhitzt, und nach 4 h wurde der vollständige Umsatz des Ausgangsmaterials festgestellt (DC). 2,5 g Celite wurden zugegeben und das Lösungsmittel am Rotationsverdampfer entfernt. Das Produkt wurde mittels zweimaliger Säulenchromatographie (1. Säule: 30 x 1,8 cm, CHCl₃/MeOH/TFA = 14:2:1, 12:2:1, 10:2:1 (Vol.), 2. Säule: 30 x 1,8 cm, CHCl₃/ MeOH/TFA = 8:1:1, 7:1:1, 6:1:1, 5:1:1, 4:1:1 (VoL)) gereinigt. Das Produkt wurde in MeOH gelöst, durch Watte filtriert und das Lösungsmittel im Vakuum entfernt. Das Produkt wurde dann in dest. H₂O (50 ml) gelöst, durch ein Celite-Kissen (2 cm) filtriert, mit 12 M HCl (500 µl) versetzt und im Vakuum getrocknet. Ausbeute: 821 mg (2,26 mmol, 78 % d. Th.) hellgelbes Pulver.

### Bezugsbeispiel 1

### Herstellung der mutierten Imin-Reduktase IR45 F190M-W191F (SEQ ID No: 3)

### A) Transformation von kompetenten BL21 (DE3)-E. coli-Zellen

Das Plasmid pET-28a(+), welches das für die Imin-Reduktase IR45 F190M-W191F kodierende Gen von *Streptomyces aurantiacus* enthält (SEQ ID No: 4), wurde von der BioCat GmbH erworben, und 2 µl davon wurden mit 100 µl chemisch kompetenten BL21(DE3)-*E. coli*-Wirtszellen in 1,5-ml-Mikrozentrifugenröhrchen vermischt. Die Gemische wurden 30 min lang auf Eis inkubiert. Danach wurden die kompetente Zellen/DNA-Gemische bei 42 °C 10 s lang schockerhitzt. 700 µl autoklaviertes LB-Medium wurden zu jeder Probe zugesetzt, gefolgt von Schütteln bei 37 °C, 300 U/min, für 1 h. Anschließend wurden 100 µl des Gemischs auf zuvor hergestellte, Kanamycin enthaltende Agarplatten aufgetragen, die über Nacht in einen Inkubator mit 37 °C gestellt wurden.

### B) Herstellung von Übernacht-Kulturen

50-ml-Plastikröhrchen wurden mit 10 ml LB-Medium gefüllt, und 10 µl Kanamycin-Lösung (50 mg/ml, Endkonzentration: 50 µg/ml) wurden zugesetzt. Eine einzelne Kultur von jeder Agarplatte wurde zur Inokulation entnommen und bei 30 °C, 120 U/min, über Nacht kultiviert.

### C) Hauptkultur

Das Enzym wurde sowohl in LB- als auch in TB-Medium hergestellt.

Für jede Charge wurden entweder 330 ml autoklaviertes LB-Medium oder 300 ml autoklaviertes TB-Medium, das mit 30 ml KH₂PO₄/K₂HPO₄-Puffer (10 ml pro 100 ml Medium) ergänzt wurde, mit jeweils 330 µl Kanamycin-Lösung (50 mg/ml, 100 µl pro 100 ml Medium) ergänzt und mit 3,3 ml der jeweiligen Übernacht-Kultur (1 ml pro 100 ml Medium) versetzt. Das Zellwachstum schritt bei 37 °C, 120 U/min fort, bis der OD₆₀₀-Wert zwischen 0,6 und 0,8 lag. Anschließend wurden die Kulturen mit IPTG (1 M Stammlösung, Endkonzentration: 0,5 mM) induziert. Die Zellkulturen wurden bei 20 °C, 120 U/min, über Nacht geschüttelt.

Zur Gewinnung der Enzyme wurden die Kulturen zentrifugiert (5000 U/min, 4 °C, 20 min), in destilliertem Wasser resuspendiert und erneut zentrifugiert (5000 U/min, 4 °C, 20 min). Die Pellets wurden in 10 ml Wasser pro Gramm feuchtem Pellet resuspendiert. Die Resuspensionen (LB- oder TB-Medium-Kultivierung) wurden entweder als ganze Zellen schockgefroren und lyophilisiert oder 5 min lang pulsierend beschallt und zentrifugiert (14000 U/min, 4 °C, 20 min). Im letzteren Fall wurden die Pellets verworfen und der Überstand schockgefroren und lyophilisiert. Die erhaltenen lyophilisierten zellfreien Extrakte (CFE) sowie ganze lyophilisierte Zellen wurden bei -20 °C gelagert.

### Beispiel 7

### Herstellung von (S)-Reticulin (S)-(15)

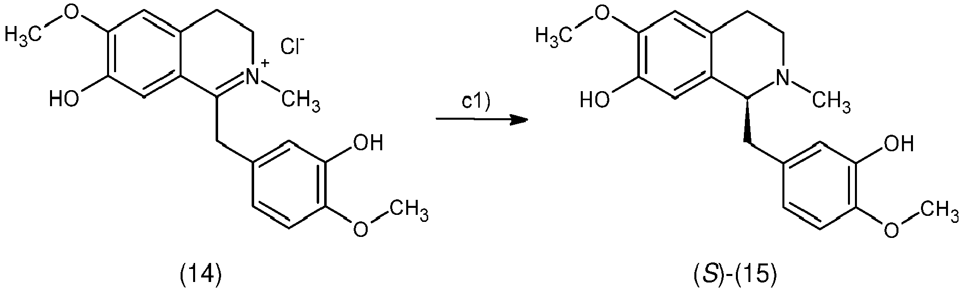

Mikrozentrifugenröhrchen (1,5 ml) wurden mit 5 mg des jeweiligen IR45 F190M-W191F-Enzym enthaltenden Präparats aus Bezugsbeispiel 1 (lyophilisierte ganze Zellen oder CFE, Endkonzentration: 20 mg/ml) und einer wässrigen Stammlösung von D-Glucose (9 mg/ml, Endkonzentration: 50 mM) beschickt. Dazu wurden eine Glucose-Dehydrogenase-Lösung (Endkonzentration: 2 mg/ml), eine Lösung von 1,2-Dehydroreticulin (14) (Endkonzentration: 10 mM), KPi-Puffer (pH 7,5, Endkonzentration: 0,1 M), NADP⁺ (20 mM-Stammlösung, Endkonzentration: 1 mM zu Beginn der Reaktion) und Wasser bis zu einem Endvolumen von 250 µl zugesetzt. Die Reaktionsgemische wurden bei 30 °C, 900 U/min, vermischt und nach 20 h durch Zusatz von 250 µl Methanol gequencht. Die Proben wurden zentrifugiert, durch Watte filtriert und mittels HPLC-UV analysiert. Die für die Ausbeuten und die Enantiomerenreinheit (Enantiomerenüberschuss, ee) gemessenen Werte der vier verschiedenen Ansätze (Kultivierung in LB- oder TB-Medium; ganze Zellen oder CFE) sind in der nachstehenden Tabelle 1 angegeben.

**Tabelle 1**

| **Medium** | **Präparat** | **Ausbeute (%)** | **ee (%)** |
|---|---|---|---|
| LB | ganze Zellen | 97,6 | 98,3 |
| LB | CFE | 95,7 | 98,6 |
| TB | ganze Zellen | 93,9 | 98,5 |
| TB | CFE | 99,9 | 98,7 |

Obwohl alle vier Enzymvarianten sehr gute Ergebnisse geliefert hatten, konnten die besten Werte somit mit dem zellfreien Extrakt von in TB-Medium kultivierten Zellen erzielt werden, der eine praktisch quantitative Ausbeute an (S)-Reticulin mit nahezu 99%iger Enantiomerenreinheit ergeben hatte. Durch eine Optimierung der Verfahrensbedingungen mittels Routineexperimenten sollten diese Ergebnisse sogar noch weiter verbesserbar und somit ein Enantiomerenüberschuss von >99,9 % erzielbar sein.

### Bezugsbeispiel 2

### Herstellung der Dehydroreticulin-Reduktase von Papaver somniferum (PsDRR) (SEQ ID No: 1)

### A) Transformation von kompetenten Arctic Express (DE3)-E. coli-Zellen

Ein das für die Dehydroreticulin-Reduktase kodierende Gen von *Papaver somniferum* enthaltendes Plasmid (von der BioCat GmbH kommerziell erworben und in pET-47b kloniert, SEQ ID No: 2; 2 µl) und 100 µl chemisch kompetente Arctic Express (DE3)-*E. coli*-Wirtszellen wurden in 1,5-ml-Mikrozentrifugenröhrchen vermischt. Die Gemische wurden 30 min lang auf Eis inkubiert. Danach wurden die kompetente Zellen/ DNA-Gemische bei 42 °C 20 s lang schockerhitzt. 700 µl autoklaviertes LB-Medium wurden zu jeder Probe zugesetzt, gefolgt von Schütteln bei 37 °C, 300 U/min, für 1 h. Anschließend wurden 100 µl des Gemischs auf zuvor hergestellte, Kanamycin enthaltende Agarplatten aufgetragen, die über Nacht in einen Inkubator mit 37 °C gestellt wurden.

### B) Herstellung von Übernacht-Kulturen

50-ml-Plastikröhrchen wurden mit 10 ml LB-Medium gefüllt, und 10 µl Kanamycin-Lösung (50 mg/ml, Endkonzentration: 50 µg/ml) wurden zugesetzt. Eine einzelne Kultur von jeder Agarplatte wurde zur Inokulation entnommen und bei 30 °C, 120 U/min, über Nacht kultiviert.

### C) Hauptkultur

Für jede Charge wurden 330 ml autoklaviertes LB-Medium mit jeweils 330 µl Kanamycin-Lösung (50 mg/ml, Endkonzentration: 50 µg/ml) ergänzt und mit 3,3 ml der jeweiligen Übernacht-Kultur (1 ml pro 100 ml Medium) versetzt. Das Zellwachstum schritt bei 30 °C, 120 U/min fort, bis der OD₆₀₀-Wert 1 betrug. Anschließend wurden die Kulturen mit IPTG (1 M Stammlösung, Endkonzentration: 1,0 mM) induziert. Die Zellkulturen wurden bei 13-15 °C, 120 U/min, 20 bis 24 h lang geschüttelt.

Zur Gewinnung der Enzyme wurden die Kulturen zentrifugiert (5000 U/min, 4 °C, 20 min), in destilliertem Wasser resuspendiert und erneut zentrifugiert (5000 U/min, 4 °C, 20 min). Die Pellets wurden in 10 ml Wasser pro Gramm feuchtem Pellet resuspendiert. Die Resuspensionen wurden entweder als ganze Zellen schockgefroren und lyophilisiert oder 5 min lang pulsierend beschallt und zentrifugiert (14000 U/min, 4 °C, 20 min). Im letzteren Fall wurden die Pellets verworfen und der Überstand schockgefroren und lyophilisiert. Die erhaltenen lyophilisierten zellfreien Extrakte (CFE) sowie ganze lyophilisierte Zellen wurden bei -20 °C gelagert.

### Beispiel 8

### Herstellung von (R)-Reticulin (R)-(15)

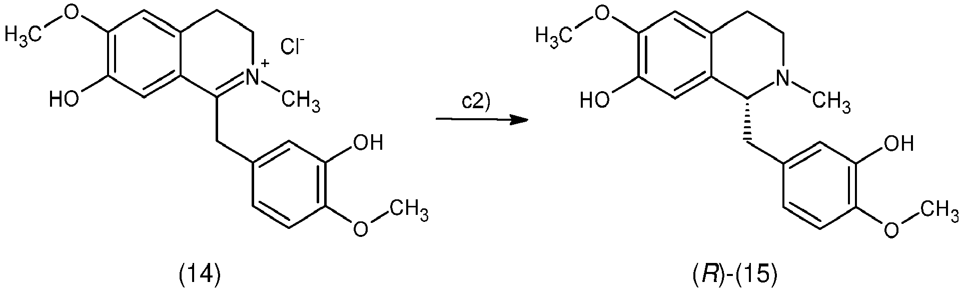

Für diese Biotransformation wurden zahlreiche Ansätze, jeweils unter Variation eines der Reaktionsparameter durchgeführt. So wurde etwa die Konzentration von 1,2-Dehydroreticulin (14) zwischen 10 und 50 mM, der pH durch die Auswahl des jeweiligen Puffers (KPi, Tris-HCl oder Glycin-NaOH) zwischen 6 und 11 sowie ein wassermischbares Co-Lösungsmittel, nämlich DMSO, EtOH, Aceton oder Isopropanol, und dessen Menge zwischen 2 und 30 Vol.-% variiert.

### Beispiel 8A - Variation der Substratkonzentration

Mikrozentrifugenröhrchen (1,5 ml) wurden mit einer wässrigen Stammlösung von D-Glucose (9 mg/ml, Endkonzentration: 50 mM) und das das PsDRR-Enzym enthaltende Präparat aus Bezugsbeispiel 2 (lyophilisierte ganze Zellen oder CFE) in Form einer Puffer-Stammlösung mit einer Konzentration von 0,1 U/ml (Endkonzentration: 0,06 U/ml) beschickt. Dazu wurden eine Glucose-Dehydrogenase-Lösung (Endkonzentration: 2 mg/ml), unterschiedliche Mengen einer Lösung von 1,2-Dehydroreticulin (14) in einem Gemisch aus Wasser und 15 Vol.-% DMSO, so dass die Endkonzentration zwischen 10 mM und 50 mM variierte, NADP⁺ (20 mM-Stammlösung, Endkonzentration: 1 mM zu Beginn der Reaktion), Tris-HCl-Puffer (pH 8,5, Endkonzentration: 0,1 M) und zusätzliches DMSO bis zu einer Endkonzentration von 10 Vol.-% zugesetzt. Die Reaktionsgemische wurden bei 30 °C, 900 U/min, vermischt und nach 20 h durch Zusatz von 400 µl Methanol gequencht. Die Proben wurden zentrifugiert, durch Watte filtriert und mittels HPLC-UV analysiert. Die Ausbeuten für die jeweilige Substratkonzentration sind in der nachstehenden Tabelle 2 angegeben.

**Tabelle 2**

| | **Konzentration 1,2-Dehydroreticulin** | | | | | |
|---|---|---|---|---|---|---|
| | 0 mM | 10 mM | 20 mM | 30 mM | 40 mM | 50 mM |
| **Ausbeute (%)** | 0 | 97,3 | 98,2 | 65,9 | 37,1 | 17,7 |

Die beste Ausbeute war demnach mit einer Konzentration des Ausgangsprodukts von 10-20 mM erzielbar.

### Beispiel 88 - Variation des pH-Werts

Mikrozentrifugenröhrchen (1,5 ml) wurden mit einer wässrigen Stammlösung von D-Glucose (9 mg/ml, Endkonzentration: 50 mM) und einer Suspension von das PsDRR-Enzym enthaltenden lyophilisierten ganzen Zellen in verschiedenen Puffern (KPi, Tris-HCl oder Glycin-NaOH) mit einer Konzentration von 0,1 U/ml (Endkonzentration: 0,06 U/ml) beschickt. Dazu wurden Glucose-Dehydrogenase-Lösung (Endkonzentration: 2 mg/ml), eine Lösung von 1,2-Dehydroreticulin (14) in einem Gemisch aus Wasser und 15 Vol.-% DMSO (Endkonzentration: 30 mM), NADP⁺-Lösung (Endkonzentration: 1 mM zu Beginn der Reaktion), eine entsprechende Menge des jeweiligen Puffers (0,1 M) zum Erhalt des gewünschten pH-Werts sowie zusätzliches DMSO bis zu einer Endkonzentration von 10 Vol.-% zugesetzt. Die Reaktionsgemische wurden bei 30 °C, 900 U/min, vermischt und nach 20 h durch Zusatz von 250 µl Methanol gequencht. Die Proben wurden zentrifugiert, durch Watte filtriert und mittels HPLC-UV analysiert. Die Ausbeuten für den jeweiligen pH-Wert und Puffer sind in der nachstehenden Tabelle 3 angegeben.

**Tabelle 3**

| **pH** | **6** | **6,5** | **7** | **7,5** | **8** | **8,5** | **9** | **9,5** | **10** | **10,5** |
|---|---|---|---|---|---|---|---|---|---|---|
| **Puffer** | **Ausbeute (%)** | | | | | | | | | |
| KPi | 66,1 | 62,1 | 76,5 | 78,1 | 76,4 | | | | | |
| Tris-HCl | | | | | 99,3 | 98,2 | 76,0 | | | |
| Glycin-NaOH | | | | | | | 81,3 | 99,0 | 99,3 | 43,5 |

Man erkennt zwei Maxima der Ausbeuten an (R)-Reticulin bei pH 8-8,5 unter Verwendung von Tris-HCl-Puffer und bei 9,5-10 unter Verwendung von Glycin-NaOH-Puffer.

### Beispiel 8C - Variation des Co-Lösungsmittels

Mikrozentrifugenröhrchen (1,5 ml) wurden mit einer wässrigen Stammlösung von D-Glucose (9 mg/ml, Endkonzentration: 50 mM) und einer Suspension von das PsDRR-Enzym enthaltenden lyophilisierten ganzen Zellen in Wasser mit einer Konzentration von 0,1 U/ml (Endkonzentration: 0,06 U/ml) beschickt. Dazu wurden eine Glucose-Dehydrogenase-Lösung (Endkonzentration: 2 mg/ml), eine Lösung von 1,2-Dehydroreticulin (14) in Wasser (Konzentration: 30 mM), variierende Mengen eines Co-Lösungsmittels (DMSO, EtOH, Aceton oder Isopropanol) zwischen 2 und 30 Vol.-%, NADP⁺-Lösung (Endkonzentration: 1 mM zu Beginn der Reaktion) und KPi-Puffer (pH 7,5, Endkonzentration: 0,1 M) zugesetzt. Die Reaktionsgemische wurden bei 30 °C, 900 U/min, vermischt und nach 20 h durch Zusatz von 250 µl Methanol gequencht. Die Proben wurden zentrifugiert, durch Watte filtriert und mittels HPLC-UV analysiert. Die Ausbeuten für die jeweilige Menge an Co-Lösungsmittels sind in der nachstehenden Tabelle 4 angegeben.

**Tabelle 4**

| **Co-Lösungsmittel** | **Konzentration (Vol./Vol.)** | **Ausbeute (%)** |
|---|---|---|
| DMSO | 0 | 33,6 |
| | 2 | 33,4 |
| | 5 | 38,6 |
| | 10 | 45,5 |
| | 15 | 32,4 |
| | 20 | 17,3 |
| | 25 | 6,0 |
| | 30 | 2,1 |
| EtOH | 2 | 32,2 |
| | 5 | 23,0 |
| | 10 | 4,8 |
| Aceton | 2 | 33,2 |
| | 5 | 18,4 |
| | 10 | 3,9 |
| Isopropanol | 2 | 31,7 |
| | 5 | 14,6 |
| | 10 | 2,6 |

Es ist zu erkennen, dass der Zusatz von 2 Vol.-% eines Co-Lösungsmittels nahezu keine Auswirkungen auf die Ausbeuten hatte und dass Letztere nur durch Zusatz von DMSO gesteigert werden konnten, wobei das Optimum zwischen 5 und 10 Vol.-% oder bei etwa 10 Vol.-% an DMSO in der wässrigen Lösung zu liegen scheint.

### Beispiel 8D - Variation der Reaktionszeit

In einer weiteren Versuchsreihe unter Einstellung einer Kombination der besten zuvor bestimmten Parameter wurde zudem die Reaktionszeit variiert, wobei sich herausstellte, dass bei Verwendung von 1,2-Dehydroreticulin (14) in einer Konzentration von 10 mM nach spätestens 4 h vollständiger Umsatz erzielt wurde.

### Beispiel 8E - optimale Bedingungen

Somit scheint die beste Kombination von Reaktionsparametern für die enantioselektive Hydrierung von 1,2-Dehydroreticulin (14) zu (R)-Reticulin (R)-(15) jene zu sein, bei der alle der folgenden Bedingungen i) bis vii) gelten:
i) 0,1 M Tris-HCl-Puffer oder Glycin-NaOH-Puffer wird als Lösungsmittel eingesetzt;
ii) 5-10 Vol.-% oder etwa 10 Vol.-% DMSO werden als Co-Lösungsmittel eingesetzt;
iii) die Dehydroreticulin-Konzentration beträgt 10-20 mM;
iv) der pH beträgt 8-8,5 (Tris-HCl) bzw. 9-9,5 (Glycin-NaOH);
v) NADPH/NADP⁺ wird als Cofaktor in einer Konzentration von 1 mM eingesetzt;
vi) Glucose-Dehydrogenase wird als Co-Enzym in einer Konzentration von 2 mg/ml und D-Glucose als Co-Substrat in einer Konzentration von 9 mg/ml eingesetzt; und
vii) die Reaktion wird 4 h lang ablaufen gelassen (für 10 mM Dehydroreticulin).

Unter Auswahl der folgenden Kombination wurde (R)-Reticulin in der Folge praktisch quantitativ und nahezu enantiomerenrein erhalten:
i) 0,1 M Tris-HCl-Puffer als Lösungsmittel;
ii) 10 Vol.-% DMSO als Co-Lösungsmittel;
iii) Dehydroreticulin-Konzentration 10 mM;
iv) pH 8;
v) 1 mM NADPH/NADP⁺;
vi) 2 mg/ml GDH und 9 mg/ml D-Glucose; und
vii) 4 h Reaktionszeit.

Als das Enzym PsDRR in Form der lyophilisierten ganzen Zellen eingesetzt wurde, konnte (R)-Reticulin in einer Ausbeute von 99,9 % d. Th. erhalten werden, und unter Verwendung des zellfreien Extrakts in einer Ausbeute von 97,5 d. Th. - und das jeweils in einem Enantiomerenüberschuss von > 99,9 % ee.

### Bezugsbeispiel 3

### Herstellung des Corytuberin-Synthase-Enzyms

Es wurden sowohl das Wildtyp-Gen von *Coptis japonica* (SEQ ID No: 28), das für die Corytuberin-Synthase (CjCorSyn, SEQ ID No: 27) kodiert, als auch ein Gen (SEQ ID No: 30), das für eine N-terminal trunkierte Version des Enzyms (NcutCorSyn, SEQ ID No: 29) kodiert, jeweils zusammen mit dem Gen (SEQ ID No: 6), das für den Redoxpartner der Corytuberin-Synthase, die Cytochrom P450-Reduktase 2 von *Arabidopsis thaliana (AtR2,* SEQ ID No: 5) kodiert, in kompetente BL21 (DE3)-E. coli-Zellen transformiert, die in der Folge die jeweiligen Enzyme heterolog exprimierten.

### A1) Transformation von kompetenten BL21 (DE3)-E. coli-Zellen mit der Reduktase AtR2

Das für die jeweilige CorSyn kodierende Gen von *Coptis japonica* und das für AtR2 kodierende Gen von *Arabidopsis thaliana* wurden jeweils in Form des Plasmids pET-28a von der BioCat GmbH erworben. Das AtR2-Gen wurde in den Vektor pCDF-1b umkloniert, um eine Co-expression der beiden Gene in zwei unterschiedlichen Plasmiden zu ermöglichen. 2 µl der Lösung des das für die AtR2-Reduktase kodierende Gen von *Arabidopsis thaliana* enthaltenden Plasmids (pCDF-1b) sowie 100 µl chemisch kompetente BL21(DE3)-*E. coli*-Zellen-Wirtszellen wurden in 1,5-ml-Mikrozentrifugenröhrchen vermischt. Die Gemische wurden 30 min lang auf Eis inkubiert. Danach wurden die kompetente Zellen/DNA-Gemische bei 42 °C 10 s lang schockerhitzt. 700 µl autoklaviertes LB-Medium wurden zu jeder Probe zugesetzt, gefolgt von Schütteln bei 37 °C, 300 U/min, für 1 h. Anschließend wurden 150 µl des Gemischs auf zuvor hergestellte, Spectinomycin enthaltende Agarplatten aufgetragen, die über Nacht in einen Inkubator mit 37 °C gestellt wurden.

### A2) Herstellung von Übernacht-Kulturen

50-ml-Plastikröhrchen wurden mit 10 ml LB-Medium gefüllt, und 50 µg/ml Spectinomycin-Lösung wurden zugesetzt. Eine einzelne Kultur von jeder Agarplatte wurde zur Inokulation entnommen und bei 37 °C, 120 U/min, über Nacht kultiviert.

### A3) Herstellung von elektrokompetenten Zellen

Es wurden 500 ml autoklaviertes LB-Medium mit 500 µl Spectinomycin-Lösung (100 mg/ml, Endkonzentration: 100 µg/ml) ergänzt und mit 5,0 ml der obigen Übernacht-Kultur (1 ml pro 100 ml Medium) versetzt. Das Zellwachstum schritt bei 37 °C, 120 U/min fort, bis der OD₆₀₀-Wert 0,5-0,7 betrug. Die Zellen wurden 20 min lang auf Eis gekühlt und in sterile Zentrifugierbecher übergeführt. Alle weiteren Schritte wurden steril möglichst bei 0 °C (auf Eis) durchgeführt.

Zur Gewinnung der Enzyme wurden die Kulturen zentrifugiert (5000 U/min, 4 °C, 15 min), der Überstand verworfen und das Zell-Pellet vorsichtig in 500 ml 10%iger wässriger Glycerin-Lösung resuspendiert. Die Zellen wurden erneut zentrifugiert (5000 U/min, 4 °C, 15 min) und das Zell-Pellet erneut in 250 ml 10%iger wässriger Glycerin-Lösung resuspendiert. Die Zellen wurden nochmals zentrifugiert (5000 U/min, 4 °C, 15 min) und das Zell-Pellet in 20 ml 10%iger wässriger Glycerin-Lösung resuspendiert. Anschließend wurde das letzte Mal zentrifugiert (5000 U/min, 4 °C, 15 min) und das Zell-Pellet in 2 ml 10%iger wässriger Glycerin-Lösung resuspendiert, in Portionen von 80 µl aliquotiert, schockgefroren und bei -80 °C gelagert.

### A4) Transformation der elektrokompetenten Zellen mit CorSyn

Ein das für die jeweilige CorSyn kodierende Gen enthaltendes Plasmid (pET-28a, 2 µl) sowie 80 µl der das *At*R2-Plasmid (in pCDF-1b) enthaltenden elektrokompetenten *BL21*(DE3)-E. coli-Wirtszellen wurden in 1,5-ml-Mikrozentrifugenröhrchen vermischt und 1 min lang inkubiert. Die jeweilige Zellsuspension wurde in eine Elektroporationsküvette übergeführt, wo sich die Zellen am Boden absetzten. Die Küvette wurde in der Elektroporationskammer platziert und mit einem MicroPulser-Elektroporator (BioRad) Elektropulsen ausgesetzt (Programm: Ec2). Die Küvette wurde anschließend aus der Kammer entfernt, der Inhalt mit 1 ml SOC-Medium versetzt und vorsichtig vermischt. Die Zellsuspension wurde in 1,5-ml-Mikrozentrifugenröhrchen übergeführt, gefolgt von Schütteln bei 37 °C, 300 U/min, für 1 h. Anschließend wurden 100 µl des Gemischs auf zuvor hergestellte, Kanamycin und Spectinomycin enthaltende Agarplatten aufgetragen, die über Nacht in einen Inkubator mit 37 °C gestellt wurden.

### B) Herstellung von Übernacht-Kulturen

50-ml-Plastikröhrchen wurden mit 10 ml LB-Medium gefüllt, und jeweils 10 µl Kanamycin-Lösung (50 mg/ml, Endkonzentration: 50 µg/ml) und Spectinomycin-Lösung (100 mg/ml, Endkonzentration: 100 µg/ml) wurden zugesetzt. Eine einzelne Kultur von jeder Agarplatte wurde zur Inokulation entnommen und bei 37 °C, 120 U/min, über Nacht kultiviert.

### C) Hauptkultur und Gewinnung der Enzyme

5 ml TB-Medium wurden mit 5 µl Kanamycin-Lösung und 5 µl Spectinomycin-Lösung (aus 50 bzw. 100 mg/ml-Stammlösungen, Endkonzentration: 50 bzw. 100 µg/ml) ergänzt und mit 1 Vol.-% der Übernacht-Kulturen in 10-ml-Deep-Well-Platten inokuliert. Die Zellen wurden bei 20 °C, 120 U/min, 9 h lang gezüchtet. Die Kulturen wurden mit 5 µl IPTG (1 mM), ergänzt mit 5 µl 5-Aminolävulinsäure (aus einer 1 M Stammlösung, Endkonzentration: 1 mM), induziert und bei 20 °C, 120 U/min, 22 h lang inkubiert. Die Kulturen wurden zentrifugiert (4000 U/min, 15 °C, 30 min) und das Pellet wurde für die Biotransformationen eingesetzt.

### Beispiel 9

### Herstellung von Corytuberin (16)

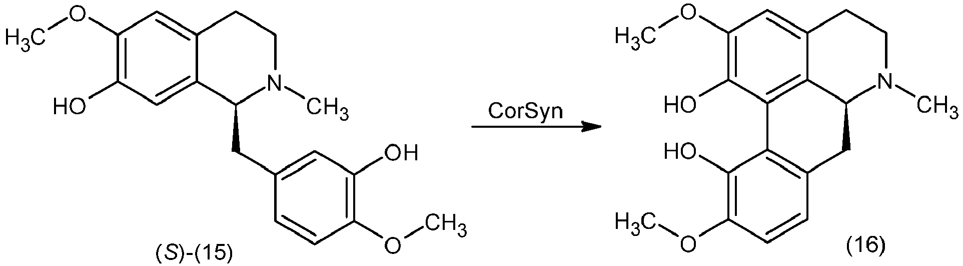

Die in Bezugsbeispiel 3 erhaltenen Pellets wurden in 0,5 ml KPi-Puffer (0,1 M, pH 7,5) resuspendiert und in 2-ml-Deep-Well-Platten übertragen, wonach das Substrat (*S*)-Reticulin (*S*)-(15) (aus einer racemischen 100 mM Stammlösung in DMSO, Endkonzentration: 0,2 mM *rac-*Reticulin) sowie NADPH-Lösung (aus 25 mM Stammlösung, Endkonzentration: 0,05 mM) und D-Glucose-Lösung (aus 1,25 M Stammlösung, Endkonzentration: 50 mM) zugesetzt wurden. Die Reaktionsgemische wurden bei 30 °C, 220 U/min, 20 h lang inkubiert. Die Proben wurden mit 200 µl Methanol gequencht und in frische Mikrozentrifugenröhrchen übergeführt. Die Proben wurden zentrifugiert (14680 U/min, 10 min) und der Überstand wurde durch Watte filtriert. Die Proben wurden mittels DC qualitativ und mittels HPLC-MS quantitativ analysiert.

Bei Verwendung der mit dem Wildtyp-Gen von *Coptis japonica* (*Cj*CorSyn) transformierten Wirtszellen wurde Corytuberin in einer Ausbeute von 14 % d. Th. erhalten, während für die mit der N-terminal trunkierten Version des Gens (NcutCorSyn) transformierten *BL21* (DE3)-Zellen eine Ausbeute von 45 % d. Th. festgestellt werden konnte. Somit ist die Verwendung von NcutCorSyn als Enzym zur Herstellung von Corytuberin aus (*S*)-Reticulin klar zu bevorzugen. Ohne sich auf eine bestimmte Theorie festlegen zu wollen, nehmen die Erfinder an, dass diese höhere Wirksamkeit auf eine stärkere Expression des trunkierten Enzyms in den Wirtszellen zurückzuführen ist.

### Bezugsbeispiel 4

### Herstellung des Salutaridin-Synthase-Enzyms

Es wurden sowohl das Wildtyp-Gen von *Papaver somniferum* (SEQ ID No: 8), das für die Salutaridin-Synthase (PsSaISyn, SEQ ID No: 7) kodiert, als auch neun weitere Gene (SEQ ID No: 10, 12, 14, 16, 18, 20, 22, 24, 26), die für verschiedene mutierte bzw. veränderte Versionen des Enzyms kodieren (SEQ ID No: 9, 11, 13, 15, 17, 19, 21, 23, 25), jeweils zusammen mit dem Gen (SEQ ID No: 6), das für den Redoxpartner der Salutaridin-Synthase, die Cytochrom P450-Reduktase 2 von *Arabidopsis thaliana* (AtR2, SEQ ID No: 5), kodiert, in kompetente BL21(DE3)- oder Arctic Express (DE3)-*E.* coli-Zellen transformiert, die in der Folge die jeweiligen Enzyme heterolog exprimierten.

Die neun anderen Versionen des Enzyms außer dem Wildtyp umfassen eine N-terminal trunkierte Version des Enzyms (NcutSalSyn, SEQ ID No: 9), eine chimäre Version davon, die am N-terminalen Ende eine Peptidsequenz eines anderen Cytochrom-P450-Enzyms, nämlich von Cheilanthifolin-Synthase CYP719A5 umfasst (CFS_PsSalSyn, SEQ ID No: 11), je eine mit Glutathion-S-Transferase- oder Small Ubiquitinlike Modifier-Markierung ergänzte Version sowohl des Wildtyps (GST-tag *Ps*SalSyn, SEQ ID No: 13; SUMO-tag *Ps*SalSyn, SEQ ID No: 17) als auch der N-terminal trunkierten Version (GST-tag NcutSalSyn, SEQ ID No: 15; SUMO-tag NcutSalSyn, SEQ ID No: 19), sowie drei Konstrukte, in denen am N-terminalen Ende der stark hydrophobe native Teil gegen 6 bis 8 Aminosäuren ausgetauscht war (NswapAL_SalSyn, SEQ ID No: 21; NswapAK_SalSyn, SEQ ID No: 23; NswapKK_SalSyn, SEQ ID No: 25). Davon wurden drei Gene (Wildtyp, SEQ ID No: 8; NcutSalSyn, SEQ ID No: 10; und CFS_PsSalSyn, SEQ ID No: 12) jeweils in Form des Plasmids pET-28a(+) von der BioCat GmbH erworben. Das Wildtyp-Gen sowie das für NcutSalSyn kodierende Gen wurden mithilfe von Restriktions-Klonierung in die Vektoren pDB-HisGST und pCIOX kloniert, die die jeweiligen Gene mit GST- oder SUMO-Tags ausstatteten. Die drei Konstrukte, in denen der N-terminale Teil des Proteins verändert war (Nswap-AL_SalSyn, NswapAK_SalSyn, NswapKK_SalSyn), wurden mittels PCR und entsprechenden Primern erzeugt.

### A1) Transformation von kompetenten BL21 (DE3)- oder Arctic Express (DE3)-E. coli-Zellen

Das für die Cytochrom-P450-Reduktase AtR2 kodierende Gen enthaltende Plasmid pCDF-1b (2 µl) sowie 100 µl chemisch kompetente *BL21* (DE3)- oder Arctic Express (DE3)-E. coli-Wirtszellen wurden in 1,5-ml-Mikrozentrifugenröhrchen vermischt. Die Gemische wurden 30 min lang auf Eis inkubiert. Danach wurden die kompetente Zellen/DNA-Gemische bei 42 °C 10 s (BL21) oder 20 s (Arctic Express) lang schockerhitzt. 700 µl autoklaviertes LB-Medium wurden zu jeder Probe zugesetzt, gefolgt von Schütteln bei 37 °C, 300 U/min, für 1 h. Anschließend wurden 150 µl des Gemischs auf zuvor hergestellte, Spectinomycin enthaltende Agarplatten aufgetragen, die über Nacht in einen Inkubator mit 37 °C gestellt wurden.

### A2) Herstellung von Übernacht-Kulturen

50-ml-Plastikröhrchen wurden mit 10 ml LB-Medium gefüllt, und 50 µg/ml Spectinomycin-Lösung wurden zugesetzt. Eine einzelne Kultur von jeder Agarplatte wurde zur Inokulation entnommen und bei 37 °C, 120 U/min, über Nacht kultiviert.

### A3) Herstellung von elektrokompetenten Zellen

Es wurden 500 ml autoklaviertes LB-Medium mit 500 µl Spectinomycin-Lösung (100 mg/ml, Endkonzentration: 100 µg/ml) ergänzt und mit jeweils 5,0 ml der obigen Übernacht-Kultur der *BL21* (DE3)- oder Arctic Express (DE3)-*E. coli*-Zellen (1 ml pro 100 ml Medium) versetzt. Das Zellwachstum schritt bei 37 °C, 120 U/min fort, bis der OD₆₀₀-Wert 0,5-0,7 betrug. Die Zellen wurden 20 min lang auf Eis gekühlt und in sterile Zentrifugierbecher übergeführt. Alle weiteren Schritte wurden steril möglichst bei 0 °C (auf Eis) durchgeführt.

Zur Gewinnung der Enzyme wurden die Kulturen zentrifugiert (5000 U/min, 4 °C, 15 min), der Überstand verworfen und das Zell-Pellet vorsichtig in 500 ml 10%iger wässriger Glycerin-Lösung resuspendiert. Die Zellen wurden erneut zentrifugiert (5000 U/min, 4 °C, 15 min) und das Zell-Pellet erneut in 250 ml 10%iger wässriger Glycerin-Lösung resuspendiert. Die Zellen wurden nochmals zentrifugiert (5000 U/min, 4 °C, 15 min) und das Zell-Pellet in 20 ml 10%iger wässriger Glycerin-Lösung resuspendiert. Anschließend wurde das letzte Mal zentrifugiert (5000 U/min, 4 °C, 15 min) und das Zell-Pellet in 2 ml 10%iger wässriger Glycerin-Lösung resuspendiert, in Portionen von 80 µl aliquotiert, schockgefroren und bei -80 °C gelagert.

### A4) Transformation der elektrokompetenten Zellen mit SalSyn

Das für die jeweilige SalSyn kodierende Gen enthaltende Plasmid pET-28a (2 µl) sowie 80 µl der das *At*R2-Plasmid (in pCDF-1b) enthaltenden elektrokompetenten BL21(DE3)- oder Arctic Express (DE3)-E. coli-Zellen wurden in 1,5-ml-Mikrozentrifugenröhrchen vermischt und 1 min lang inkubiert. Die jeweilige Zellsuspension wurde in eine Elektroporationsküvette übergeführt, in der sich die Zellen am Boden absetzten. Die Küvette wurde in der Elektroporationskammer platziert und mit einem MicroPulser-Elektroporator (BioRad) Elektropulsen ausgesetzt (Programm: Ec2). Die Küvette wurde anschließend aus der Kammer entfernt, der Inhalt mit 1 ml SOC-Medium versetzt und vorsichtig vermischt. Die Zellsuspension wurde in 1,5-ml-Mikrozentrifugenröhrchen übergeführt, gefolgt von Schütteln bei 37 °C, 300 U/min, für 1 h. Anschließend wurden 100 µl des Gemischs auf zuvor hergestellte, Kanamycin und Spectinomycin enthaltende Agarplatten aufgetragen, die über Nacht in einen Inkubator mit 37 °C gestellt wurden.

### B) Herstellung von Übernacht-Kulturen

50-ml-Plastikröhrchen wurden mit 10 ml LB-Medium gefüllt, und 50 µg/ml Kanamycin und 100 µg/ml Spectinomycin wurden zugesetzt. Eine einzelne Kultur von jeder Agarplatte wurde zur Inokulation entnommen und bei 37 °C, 120 U/min, über Nacht kultiviert.

### C) Hauptkultur und Gewinnung der Enzyme

5 ml TB-Medium wurden mit 5 µl Kanamycin-Lösung und 5 µl Spectinomycin-Lösung (aus 50 bzw. 100 mg/ml-Stammlösungen, Endkonzentration: 50 bzw. 100 µg/ml) ergänzt und mit 1 Vol.-% der Übernacht-Kulturen in 10-ml-Deep-Well-Platten inokuliert. Die Zellen wurden, je nach Ansatz und den Wirtszellen, BL21(DE3)- oder Arctic Express (DE3)-E. coli-Zellen, bei 20 °C, 30 °C bzw. 37 °C, 120 U/min, 9 bzw. 6 h lang inkubiert. Die Kulturen wurden mit 5 µl IPTG (1 mM), ergänzt mit 5 µl 5-Aminolävulinsäure (aus einer 1 M Stammlösung, Endkonzentration: 1 mM), induziert und bei 20 °C, 120 U/min, 22 h lang inkubiert. Die Kulturen wurden zentrifugiert (4000 U/min, 15 °C, 30 min) und das Pellet wurde für die Biotransformationen eingesetzt.

### Beispiel 10

### Herstellung von Salutaridin (17)

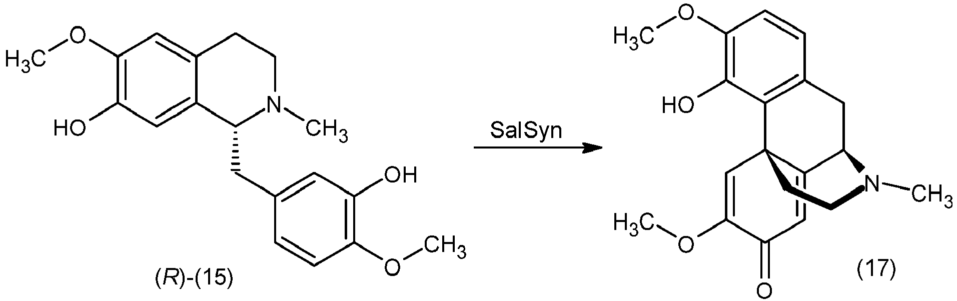

Die in Bezugsbeispiel 4 erhaltenen Pellets wurden in 0,5 ml KPi-Puffer (0,1 M, pH 7,5) resuspendiert und in 2-ml-Deep-Well-Platten übertragen, wonach das Substrat (R)-Reticulin (R)-(15) (aus einer racemischen 100 mM Stammlösung in DMSO, Endkonzentration: 0,2 mM rac-Reticulin) sowie NADPH-Lösung (aus 25 mM Stammlösung, Endkonzentration: 0,05 mM) und D-Glucose-Lösung (aus 1,25 M Stammlösung, Endkonzentration: 50 mM) zugesetzt wurden. Die Reaktionsgemische wurden bei 30 °C, 220 U/min, 20 h lang inkubiert. Die Proben wurden mit 200 µl Methanol gequencht und in frische Mikrozentrifugenröhrchen übergeführt. Die Proben wurden zentrifugiert (14680 U/min, 10 min) und der Überstand wurde durch Watte filtriert. Die Proben wurden mittels HPLC-MS, HR-MS und DC qualitativ und mittels HPLC-UV quantitativ analysiert.

In der nachstehenden Tabelle 5 sind die Ergebnisse der Ausbeuten für die in den jeweiligen E. coli-Wirtszellkulturen, *BL21* (DE3) ("BL21") oder Arctic Express (DE3) ("AE") produzierten zehn verschiedenen Enzymkombinationen zusammen mit der Inkubationstemperatur und -dauer für die Zellvermehrung bzw. die Expression angegeben.

**Tabelle 5**

| Wirtszelle | Enzym | Zellvermehrung | | Expression | | Ausbeute (%) |
|---|---|---|---|---|---|---|
| | | Temp. (°C) | Zeit (h) | Temp. (°C) | Zeit (h) | |
| BL21 | NcutSalSyn, SEQ ID No: 9 | 20 | 9 | 20 | 22 | 43,4 |
| | | 37 | 6 | 20 | 22 | 17 |
| BL21 | *Ps*SalSyn, SEQ ID No: 7 | 20 | 9 | 20 | 22 | 58,1 |
| | | 37 | 6 | 20 | 22 | 0 |
| BL21 | CFS_*Ps*SalSyn, SEQ ID No: 11 | 20 | 9 | 20 | 22 | 85,2 |
| | | 37 | 6 | 20 | 22 | 98 |
| AE | GST-tag NcutSalSyn SEQ ID No: 15 | 20 | 9 | 13 | 22 | 29,8 |
| | | 30 | 6 | 13 | 22 | 2 |
| AE | GST-tag *Ps*SalSyn, SEQ ID No: 13 | 20 | 9 | 13 | 22 | 6,3 |
| | | 30 | 6 | 13 | 22 | 4 |
| AE | SUMO-tag NcutSalSyn, SEQ ID No: 19 | 20 | 9 | 13 | 22 | 82,8 |
| | | 30 | 6 | 13 | 22 | 26 |
| AE | SUMO-tag *Ps*SalSyn, SEQ ID No: 17 | 20 | 9 | 13 | 22 | 9,9 |
| | | 30 | 6 | 13 | 22 | 4 |
| BL21 | NswapAL_SalSyn, SEQ ID No: 21 | 20 | 9 | 20 | 22 | 0,0 |
| | | 37 | 6 | 20 | 22 | 3 |
| BL21 | NswapAK_SalSyn, SEQ ID No: 23 | 20 | 9 | 20 | 22 | 28,2 |
| | | 37 | 6 | 20 | 22 | 16 |
| BL21 | NswapKK_SalSyn, SEQ ID No: 25 | 20 | 9 | 20 | 22 | 100 |
| | | 37 | 6 | 20 | 22 | 65 |

Man erkennt, dass die Wirksamkeit der jeweiligen Salutaridin-Synthase in Abhängigkeit von der Wirtszelle und den Expressions- und Kultivierungsbedingungen stark variiert. Dennoch geht aus der Tabelle auch klar hervor, dass bei geeigneter Wahl der Bedingungen Salutaridin in quantitativer Ausbeute erhalten werden konnte, wobei die Enzyme mit den besten Ausbeuten CFS_*Ps*SalSyn und NswapKK_SalSyn waren.

Die vorliegende Erfindung stellt somit ein Verfahren bereit, durch das die Alkaloide Corytuberin und Salutaridin mittels weniger Reaktionsschritte in sehr guten Ausbeuten erhalten werden können, was nach dem Stand der Technik bislang nicht möglich war.

## Patentansprüche

1. Verfahren zur Herstellung von Corytuberin und Salutaridin über Reticulin als Zwischenprodukt, wobei das Verfahren die folgenden Reaktionsschritte umfasst:
a) Amidierung von 2-(4-Hydroxy-3-methoxyphenyl)-N-methylethanamin (12) mit 3-Hydroxy-4-methoxyphenylessigsäure zum N-[2-(4-Hydroxy-3-methoxyphenyl)ethyl-N-methyl-3-hydroxy-4-methoxyphenylessigsäureamid (13): 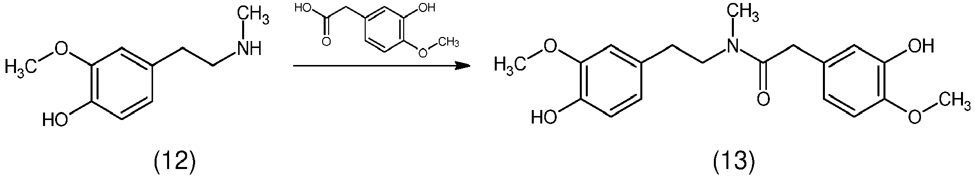
b) Bischler-Napieralski-Zyklisierung des tertiären Amids (13) zu 1,2-Dehydroreticulin (14): 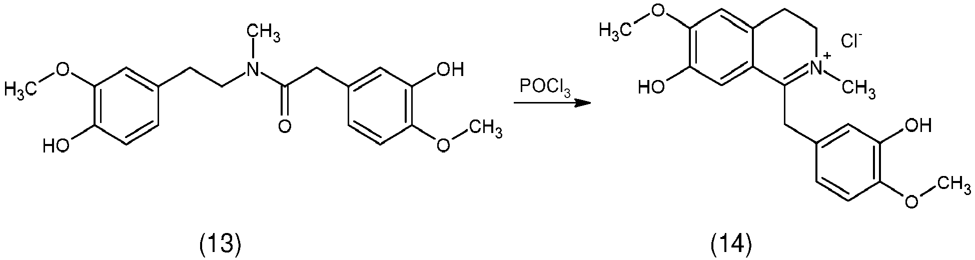
c) biokatalytische enantioselektive Hydrierung von 1,2-Dehydroreticulin (14) zu Reticulin (15) und zwar entweder
c1) unter Verwendung einer Imin-Reduktase zu (*S*)-Reticulin (*S*)-(15) oder
c2) unter Verwendung einer Dehydroreticulin-Reduktase zu (*R*)-Reticulin (*R*)-(15): 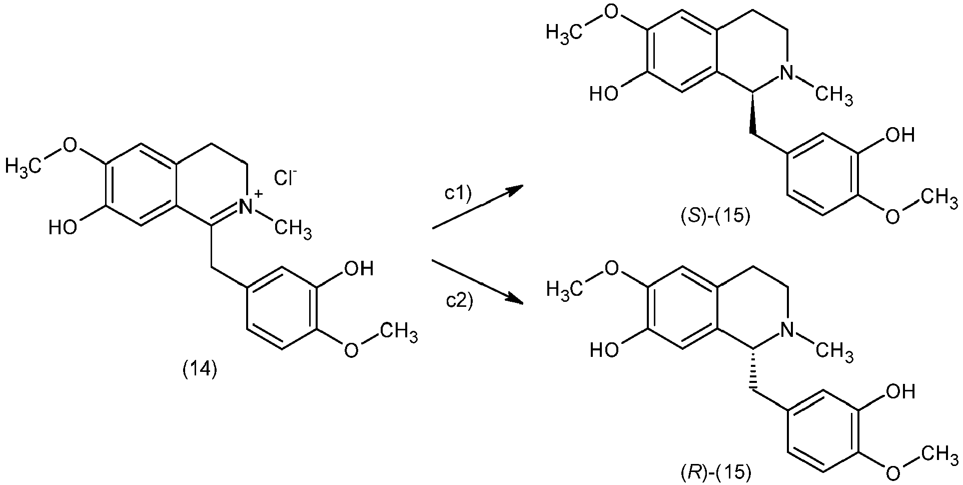
d) biokatalytische oxidative Phenol-Kupplung zwischen den beiden aromatischen Ringen des jeweiligen Reticulins und zwar entweder
d1) ortho-ortho-Kupplung von (*S*)-Reticulin in Gegenwart von Corytuberin-Synthase (CorSyn) zu Corytuberin (16) oder
d2) ortho-para-Kupplung von (*R*)-Reticulin in Gegenwart von Salutaridin-Synthase (SalSyn) zu Salutaridin (17):

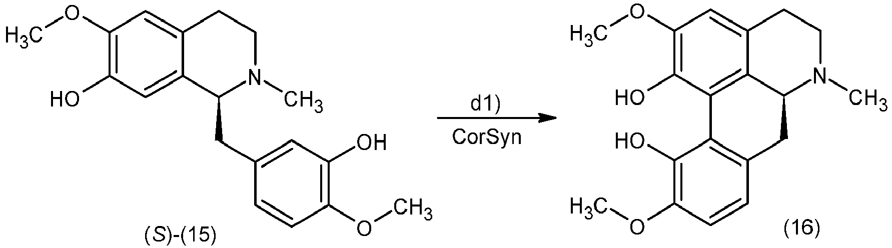

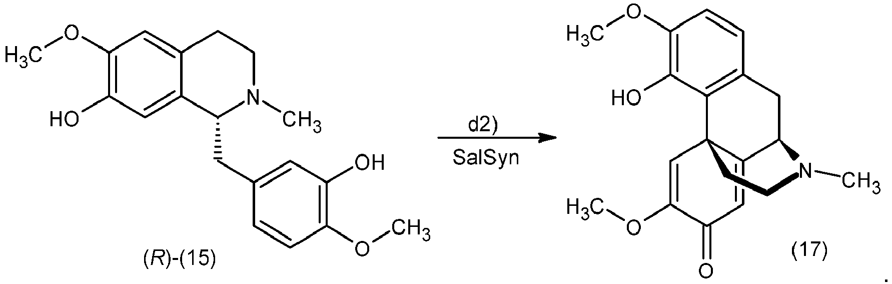

2. Verfahren nach Anspruch 1, **dadurch gekennzeichnet, dass** die Amidierung in Schritt a) unter Verwendung eines Carbodiimids durchgeführt wird,
wobei als Carbodiimid gegebenenfalls 1-Ethyl-3-(3-dimethylaminopropyl)carbodiimid (EDC) eingesetzt wird
wobei die Amidierung gegebenenfalls in Gegenwart von Hydroxyiminocyanessigsäureethylester (Oxyma) als Additiv durchgeführt wird.

3. Verfahren nach Anspruch 1 oder 2, **dadurch gekennzeichnet, dass** die Bischler-Napieralski-Zyklisierung in Schritt b) in Acetonitril als Lösungsmittel, gegebenenfalls unter Rückfluss, durchgeführt wird.

4. Verfahren nach einem der Ansprüche 1 bis 3, **dadurch gekennzeichnet, dass** die biokatalytische enantioselektive Hydrierung in Schritt c1) unter Verwendung der mutierten Imin-Reduktase IR45 F190M-W191F durchgeführt wird,
wobei gegebenenfalls ein von *E.* coli-Zellen, gegebenenfalls von kompetenten BL21(DE3)-Zellen, heterolog exprimiertes rekombinantes Enzym in Form der lyophilisierten Zellen oder eines zellfreien Extrakts davon eingesetzt wird.

5. Verfahren nach Anspruch 4, wobei die Hydrierung in Schritt c1) unter einer oder mehreren oder allen der folgenden Bedingungen i) bis v) durchgeführt wird:
i) 0,1 M KPi-Puffer wird als Lösungsmittel eingesetzt;
ii) die Dehydroreticulin-Konzentration beträgt 10-30 mM, gegebenenfalls 10-20 mM, gegebenenfalls 10 mM;
iii) der pH beträgt 7-8,5, gegebenenfalls etwa 7,5;
iv) NADPH/NADP⁺ wird als Cofaktor, gegebenenfalls in einer Konzentration von 1 mM, eingesetzt;
v) Glucose-Dehydrogenase wird als Co-Enzym, gegebenenfalls in einer Konzentration von 2 mg/ml, und D-Glucose als Co-Substrat, gegebenenfalls in einer Konzentration von 9 mg/ml, eingesetzt.

6. Verfahren nach einem der Ansprüche 1 bis 3, **dadurch gekennzeichnet, dass** die biokatalytische enantioselektive Hydrierung in Schritt c2) unter Verwendung der Dehydroreticulin-Reduktase von *Papaver somniferum* (*Ps*DRR) durchgeführt wird,
wobei gegebenenfalls ein von *E. coli*-Zellen, gegebenenfalls von kompetenten Arctic Express (DE3)-Zellen, heterolog exprimiertes rekombinantes Enzym in Form der lyophilisierten Zellen oder eines zellfreien Extrakts davon eingesetzt wird.

7. Verfahren nach Anspruch 6, **dadurch gekennzeichnet, dass** die biokatalytische enantioselektive Hydrierung in Schritt c2) unter einer oder mehreren oder allen der folgenden Bedingungen i) bis vi) durchgeführt wird:
i) 0,1 M Tris-HCl-Puffer oder Glycin-NaOH-Puffer wird als Lösungsmittel eingesetzt;
ii) 5-10 Vol.-% DMSO werden als Co-Lösungsmittel eingesetzt;
iii) die Dehydroreticulin-Konzentration beträgt 10-30 mM, gegebenenfalls 10-20 mM;
iv) der pH beträgt 8-8,5 bzw. 9-9,5;
v) NADPH/NADP⁺ wird als Cofaktor, gegebenenfalls in einer Konzentration von 1 mM, eingesetzt;
vi) Glucose-Dehydrogenase wird als Co-Enzym, gegebenenfalls in einer Konzentration von 2 mg/ml, und D-Glucose als Co-Substrat, gegebenenfalls in einer Konzentration von 9 mg/ml, eingesetzt.

8. Verfahren nach einem der Ansprüche 1 bis 5, **dadurch gekennzeichnet, dass** die biokatalytische oxidative Phenol-Kupplung zwischen den beiden aromatischen Ringen des (S)-Reticulins in Schritt d1) unter Verwendung einer Corytuberin-Synthase, gegebenenfalls der Corytuberin-Synthase von *Coptis japonica* (*Cj*CorSyn) oder einer modifizierten Version davon, zusammen mit einer Cytochrom P450-Reduktase, gegebenenfalls der Cytochrom P450-Reduktase 2 von *Arabidopsis thaliana* (*At*R2)*,* als Redoxpartner durchgeführt wird,
wobei gegebenenfalls die Corytuberin-Synthase und die AtR2 als von *E. coli-*Zellen, gegebenenfalls von kompetenten BL21 (DE3)-Zellen, heterolog exprimierte rekombinante Enzyme in Form der ganzen Zellen oder eines zellfreien Extrakts davon eingesetzt werden.

9. Verfahren nach Anspruch 8, **dadurch gekennzeichnet, dass** die ortho-ortho-Kupplung von (S)-Reticulin in Schritt d1) unter einer oder mehreren oder allen der folgenden Bedingungen i) bis vii) durchgeführt wird:
i) 0,1 M KPi-Puffer wird als Lösungsmittel eingesetzt;
ii) die Enzyme werden in Form von resuspendierten ganzen Zellen eingesetzt;
iii) 0,2-10 Vol.-% DMSO werden als Co-Lösungsmittel eingesetzt;
iv) die Reticulin-Konzentration beträgt 0,1-5 mM;
v) der pH beträgt 7,5-8,0;
vi) NADPH/NADP⁺ wird als Cofaktor, gegebenenfalls in einer Konzentration von 0,05 mM, eingesetzt;
vii) ein Glucose-Dehydrogenase-Präparat wird als Co-Enzym, gegebenenfalls in einer Konzentration von 2 mg/ml, und D-Glucose als Co-Substrat, gegebenenfalls in einer Konzentration von 50 mM, eingesetzt.

10. Verfahren nach einem der Ansprüche 1 bis 3, Anspruch 6 oder Anspruch 7, **dadurch gekennzeichnet, dass** die biokatalytische oxidative Phenol-Kupplung zwischen den beiden aromatischen Ringen des (R)-Reticulins in Schritt d2) unter Verwendung einer Salutaridin-Synthase, gegebenenfalls der Salutaridin-Synthase von *Papaver somniferum* (PsSalSyn), einer chimären Version davon (CFS_*Ps*SalSyn) oder einem Konstrukt davon (NswapKK_SalSyn), zusammen mit einer Cytochrom P450-Reduktase, gegebenenfalls der Cytochrom P450-Reduktase 2 von *Arabidopsis thaliana (AtR2),* als Redoxpartner durchgeführt wird,
wobei gegebenenfalls die Salutaridin-Synthase und die AtR2 als von *E. coli-*Zellen, gegebenenfalls von kompetenten BL21(DE3)-Zellen, heterolog exprimierte rekombinante Enzyme in Form der ganzen Zellen oder eines zellfreien Extrakts davon eingesetzt werden.

11. Verfahren nach Anspruch 10, **dadurch gekennzeichnet, dass** die ortho-para-Kupplung von (R)-Reticulin in Schritt d2) unter einer oder mehreren oder allen der folgenden Bedingungen i) bis vii) durchgeführt wird:
i) 0,1 M KPi-Puffer wird als Lösungsmittel eingesetzt;
ii) die Enzyme werden in Form von resuspendierten ganzen Zellen eingesetzt;
iii) 0,2-2 Vol.-% DMSO werden als Co-Lösungsmittel eingesetzt;
iv) die Reticulin-Konzentration beträgt 0,1-0,5 mM;
v) der pH beträgt 7,5-8,0;
vi) NADPH/NADP⁺ wird als Cofaktor, gegebenenfalls in einer Konzentration von 0,05 mM, eingesetzt;
vii) ein Glucose-Dehydrogenase-Präparat wird als Co-Enzym, gegebenenfalls in einer Konzentration von 2 mg/ml, und D-Glucose als Co-Substrat, gegebenenfalls in einer Konzentration von 50 mM, eingesetzt.

12. Verfahren nach einem der vorangegangenen Ansprüche, **dadurch gekennzeichnet, dass** das in Schritt a) eingesetzte Ausgangsprodukt 2-(4-Hydroxy-3-methoxyphenyl)-N-methylethanamin (12) zuvor ausgehend von 4-Allyl-2-methoxyphenol (Eugenol) durch die folgenden Reaktionsschritte hergestellt wird:
I) Ozonolyse von 4-Allyl-2-methoxyphenol (9) zu 4-Hydroxy-3-methoxyphenyl-acetaldehyd (Homovanillin) (10): 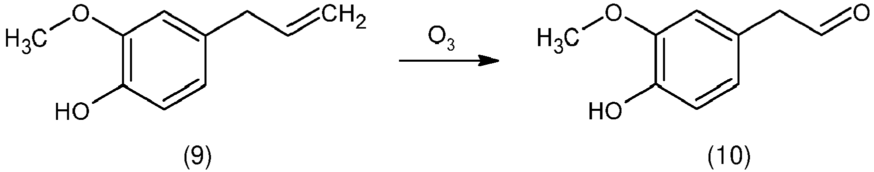
II) reduktive Aminierung des Aldehyds (10) mit N-Benzylmethanamin zum Benzyl-geschützten Zwischenprodukt N-Benzyl-2-(4-hydroxy-3-methoxyphenyl)-N-methylethanamin (11): 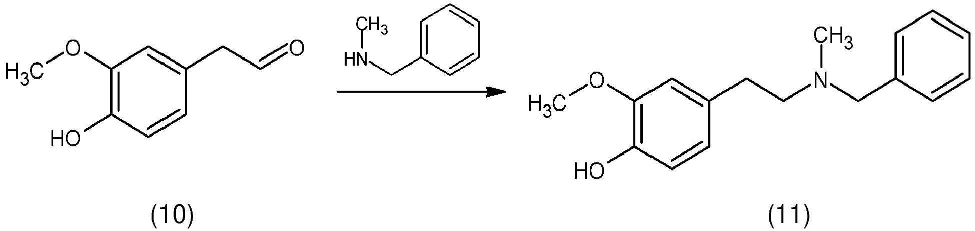
III) Hydrogenolyse zur Entfernung der Benzyl-Schutzgruppe, um das gewünschte Amin (12) zu erhalten:

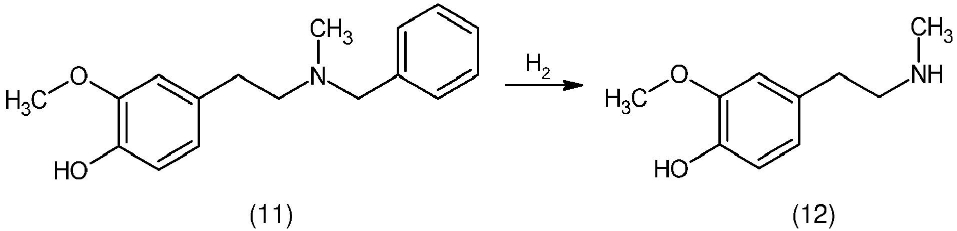

13. Verfahren nach Anspruch 12, **dadurch gekennzeichnet, dass**
die Ozonolyse von Eugenol (9) in Schritt I) in organischer Lösung durch Einleiten eines Gemischs aus O₃/O₂ bei einer Temperatur < 0 °C, gegebenenfalls -15 °C, und anschließendes Quenchen mit Dimethylsulfid durchgeführt wird; und/oder
die reduktive Aminierung des Aldehyds (10) in Schritt II) in organischer Lösung in Gegenwart eines Borhydrids, gegebenenfalls von NaBH(OAc)₃, unter Eisbadkühlung durchgeführt wird; und/oder
die Hydrogenolyse in Schritt III) in alkoholischer Lösung in Gegenwart eines Palladium-Katalysators, gegebenenfalls von Pd/C, und von HCl bei Raumtemperatur durchgeführt wird.

14. Verfahren nach Anspruch 12 oder 13, **dadurch gekennzeichnet, dass** die Schritte I) und II) als Eintopfreaktion durchgeführt werden.
